# EUROPEAN PATENT APPLICATION

(11) **EP 1 891 981 A2**
(43) Date of publication of application: **27.02.2008**
(21) Application number: 07016288.8
(22) Date of filing: 20.08.2007
(51) Int. Cl.: A61L 9/14, F24F 3/16

(54) **Air conditioner, air conditioning system, air filtering apparatus and air filtering system**

(30) Priority: 25.08.2006 JP 2006228753; 28.08.2006 JP 2006230103; 28.08.2006 JP 2006230104; 28.08.2006 JP 2006230242; 28.08.2006 JP 2006230243
(71) Applicant: Sanyo Electric Co., Ltd., Moriguchi-shi, Osaka-fu (JP)
(72) Inventor: Takahashi, Kazuo, Ota-shi Gunma 373-0036 (JP); Uchida, Yoichi, Ashikaga-shi Tochigi 326-0006 (JP); Usui, Hiroaki, Ora-gun Gunma 370-0523 (JP); Ogura, Nobuhiro, Kiryu-shi Gunma 376-0034 (JP); Nagae, Koji, Ora-gun Gunma 370-0514 (JP); Yamamoto, Tetsuya, Ota-sgi Gunma 373-0806 (JP); Rakuma, Tsuyoshi, Ora-gun Gunma 370-0524 (JP); Kurokawa, Keiko, Ora-gun Gunma 370-0517 (JP); Suzuki, Daisuke, Ota-shi Gunma 373-0817 (JP); Kuribara, Hiroyuki, Ota-shi Gunma 373-0817 (JP); Fukushima, Toshio, Ota-shi Gunma 373-0038 (JP); Nishihara, Takuro, Ota-shi Gunma 373-0817 (JP)
(74) Representative: Glawe, Delfs, Moll

(57) **Abstract**

An air conditioner having a refrigerant circuit including a compressor, a four-way valve, an outdoor heat exchanger, an air blower for sucking air and blowing out the air to a room, an indoor heat exchanger for heat-exchanging the air sucked by the air blower, an air filtering unit for bringing the air sucked by the air blower into contact with electrolytic water containing active oxygen species sucked to filter the air, and an electrolytic water supply unit for electrolyzing water containing predetermined ion species to generate electrolytic water containing active oxygen species of a prescribed concentration and supplying the generated electrolytic water to the air filtering unit. The indoor heat exchanger, the air blower and the air filtering unit are disposed in the housing of an indoor unit.

## Description

### BACKGROUND OF THE INVENTION

### 1. Field of the Invention

The present invention relates to an air conditioner, an air conditioning system, an air filtering apparatus and an air filtering system that canremovemicroorganisms (bacteria, virus, fungus, etc. (hereinafter referred to as "virus, etc.") floating in the air.

### 2. Description of the Related Art

There has been proposed an air filtering apparatus including an electrolytic water supply unit for generating electrolytic water containing active oxygen species such as hypochlorous acid or the like by electrolyzing tap water or the like, and an air filter ing unit to which the generated electrolytic water. In this air filtering apparatus, air is supplied to the air filtering apparatus and brought into contact with the electrolytic water to decompose, remove, etc. harmful components (for example, virus, bacteria, fungus, etc.) contained in the air (applications associated with this air filtering apparatus have been already filed by the applicant of this application, but have not yet been published). Furthermore, there is also known an air cleaning method for an air conditioner to which an air cleaner for oxidizing, decomposing and removing harmful materials from sucked air is connected. According to this method, air is once sucked from a room and filtered to oxidize, decompose and remove harmful materials from the air, and then returned to the air conditioner. The filtered air is air-conditioned in the air conditioner and then clean air is blown out to the room (for example, JP-A-2003-250876).

Furthermore, there is also known an air conditioner equipped with a humidifier in an indoor unit to supply comfortable air into a room (for example, Japanese Patent No. 2,714,076). This humidifier is equipped with a hydrophilic humidifying element. Water is supplied to the humidifying element and air to be air-conditioned is passed through the humidifying element, whereby the air is humidified. In general, an air conditioner is equipped with a heat exchanger for air-conditioning air sucked by an air blower, and a drain pan is disposed below the heat exchanger. The drain pan receives dew condensation water dropped from the heat exchanger, and the dew condensation water is stocked as drain water in the drain pan. This drain is properly discharged through a drain pipe (for example, JP-A-2004-93005).

Scales occurring through the electrolysis (for examples, calcium-based scales such as calcium carbonate, magnesium-based scales such as magnesium carbonate, etc.) adhere to electrodes in the electrolytic water supply unit, and thus scales adhere to the air filtering unit, so that the air filtering unit is stained. Furthermore, the humidifying element provided in an indoor unit is stained in accordance with the operation style. However, in order to clean the humidifier, a user or maintenance person must detach the humidifier from the indoor unit. Particularly, when the indoor unit is embedded in the ceiling, the work of detaching the humidifier is arduous labors, and thus much labor is needed to cleaning. Still furthermore, various bacteria, fungus, etc. are liable to breed in the drain pan because water is stocked in the drain pan, and thus there is a risk that microorganisms such as bacteria, virus, fungus, etc. (hereinafter referred to as "virus, etc.") occurring in the drain pan may be blown to a room under air condition together with air.

Furthermore, water such as tap water or the like to be used for electrolysis contains ion species such as chloride ions (chlorine ions) or the like, and the concentration of the ion species may be varied in accordance with temperature, district or season. Furthermore, natural water such as well water or the like frequently contains a low concentration of ion species. When the concentration of chloride contained in water supplied from a water supply source is low, electrolytic water cannot be efficiently generated.

Still furthermore, when the concentration of active oxygen species required for air filtering in electrolytic water (for example, about 2 to 10ppm in the case of hypochlorous acid). The concentration of the active oxygen species is preferably adjusted to a proper value in accordance with the harmful components to be filtered. Here, in order to adjust the concentration of the active oxygen species contained in electrolytic water to a predetermined value, it is required to control the electrolysis condition in accordance with the water quality (for example, the concentration of chloride ions) of tap water or the like supplied for electrolysis. However, the concentration of active oxygen species is low, and the water quality of tap water or the like is varied in accordance with season, temperature, district, etc., and thus it is complicated to strictly control the electrolysis condition in accordance with the variation of the water quality of tap water or the like for electrolysis and thus adjust the concentration of active oxygen species to a predetermined concentration, and thus the efficiency of generating electrolytic water is limited.

### SUMMARY OF THE INVENTION

The present invention has been implemented in view of the foregoing situation, and has an object to provide an air conditioner containing an air filtering apparatus in which an air filtering unit can be prevented from being stained with scales removed in an electrolytic water supply unit.

Another object of the present invention is to provide an air conditioner in which the air filtering unit can be easily cleaned.

Further object of the present invention is to provide an air conditioner in which breeding of various bacterial, virus, fungus, etc. in a drain pan can be prevented.

Further object of the present invention is to provide an air conditioner, an air conditioning system, an air filtering apparatus and an air filtering system in which electrolytic water can be generated efficiently and also the concentration of active oxygen species in the electrolytic water can be easily adjusted.

Further object of the present invention is to provide an air conditioner, an air conditioning system, an air filtering apparatus and an air filtering system in which electrolytic water containing active oxygen species can be efficiently generated irrespective of the concentration of ion species of water supplied from a water supply source, whereby air can be filtered by the electrolytic water.

In order to attain the above objects, according to a first aspect of the present invention, there is provided an air conditioner having a refrigerant circuit including a compressor, a four-way valve, an outdoor heat exchanger, an air blower for sucking air and blowing out the air to a room, and an indoor heat exchanger for heat-exchanging the air sucked by the air blower, the indoor heat exchanger and the air blower being equipped in a housing of an indoor unit, characterized by comprising: an air filtering unit that is disposed in the indoor unit and brings the air sucked by the air blower into contact with electrolytic water containing active oxygen species sucked to filter the air, and an electrolytic water supply unit for electrolyzing water containing predetermined ion species to generate electrolytic water containing active oxygen species of a prescribed concentration and supplying the generated electrolytic water to the air filtering unit.

In the above air conditioner, it is preferable that the indoor unit further includes a bypass flow passage that is disposed between the air filtering unit and the electrolytic water supply unit and through which foreign materials from the electrolytic water supply unit are selectively removed.

In the above air conditioner, it is preferable that the foreign materials are scales occurring due to generation of the electrolytic water.

In the above air conditioner, it is preferable that the indoor unit includes a main flow passage through which the electrolytic water is supplied from the electrolytic water supply unit to the air filtering unit, and the bypass flow passage is branched from the main flow passage at some position between the air filtering unit and the electrolytic water supply unit.

In the above air conditioner, it is preferable that the electrolytic water supply passage is equipped with an opening/closing valve that is closed to prevent the foreign materials from flowing through the electrolytic water supply passage to the air filtering unit when the foreign materials are removed, and the bypass flow passage is equipped with an opening/closing valve that is closed to prevent the electrolytic water from flowing through the bypass flowpassage when the foreign materials are not removed.

In the above air conditioner, it is preferable that the indoor unit further includes a drain pan disposed below the heat exchanger and a water receiving tray disposed below the air filtering unit, and the bypass flow passage is connected to at least one of the drain pan and the water receiving tray to discharge the foreign materials from the electrolytic water supply unit to at least one of the drain pan and the water receiving tray.

In the above air conditioner, it is preferable that the electrolytic water supply unit has at least one pair of electrodes for electrolyzing water, and scales occurring on the electrodes through the electrolysis of water are removed by inverting the polarities of the electrodes.

It is preferable that the above air conditioner further comprises a controller for controlling a process of generating the electrolytic water in the electrolytic water supply unit, and on the basis of a control signal from the controller, the electrolyticwatersupplyunitgenerateselectrolyticwaterwhose concentration is higher than that of the electrolytic water used for air filtering, and supplies the higher-concentration electrolytic water to the air filtering unit.

In the above air conditioner, it is preferable that the higher-concentration electric water is supplied from the electrolytic water supply unit to the air filtering unit under the state that the operation of the air blower is stopped.

In the above air conditioner, it is preferable a cleaning operation mode is provided as one of operation modes of the indoor unit, and the controller controls the electrolytic water supply unit to supply the higher-concentration electrolytic water to the air filtering unit for a predetermined time or at a predetermined time interval in the cleaning operation mode.

In the above air conditioner, it is preferable that the cleaning operation mode is a mode in which the higher-concentration electrolytic water is supplied to the air filtering unit at the predetermined time interval while the operation of the indoor unit is stopped.

In the above air conditioner, it is preferable that the cleaning operation mode is a mode in which the higher-concentration electrolytic water is supplied to the air filtering unit for the predetermined time while the indoor unit is continuously operated.

In the above air conditioner, it is preferable that the controller controls the electrolytic water supply unit to change a current value for electrolysis, thereby adjusting the concentration of the electrolytic water determined on the basis of the current value.

In the above air conditioner, it is preferable that the air filtering unit is equipped with a gas-liquid contact member at which the indoor air and the electrolytic water are brought into contact with each other, and a water receiving tray for temporarily stocking the electrolytic water dropped from the gas-liquid contact member, and a circulating pump for pumping up the electrolytic water stocked in the water receiving tray and circulating the electrolytic water concerned to the gas-liquid contact member.

It is preferable that the above air conditioner further comprises a drain pan that is disposed below the heat exchanger and stocks drain water dropped from the heat exchanger, and a passage through which the electrolytic water stocked in the water receiving tray is introduced from the water receiving tray to the drain pan. 16.

It is preferable that the above air conditioner further comprises an electromagnetic valve that is provided in the passage and opened/closed to adjust the amount of the electrolytic water to be introduced from the water receiving tray to the drain pan.

In the above air conditioner, it is preferable that the electromagnetic valve is intermittently opened/closed to thereby intermittently introduce the electrolytic water stocked in the water receiving tray to the drain pan.

It is preferable that the above air conditioner further comprises a dam portion provided in the passage, wherein electrolytic water flowing over the dam portion is introduced to the drain pan.

It is preferable that the air conditioner further comprises a water level detecting unit for detecting whether the electrolytic water stocked in the electrolytic receiving tray is equal to a predetermined water level or less, and a water supply unit for supplying one of water or water containing the predetermined ion species.

In the above air conditioner, it is preferable that the predetermined ion species is chlorine ion.

It is preferable that the air conditioner further comprises an ion species adding unit for adding the predetermined ion species to water supplied from an external water supply source to the electrolytic water supply unit.

In the above air conditioner, it is preferable that the ion species adding unit is disposed in a water distributing passage of a water distributing pipe through which the electrolytic water supply unit and an external water supply source are connected to each other, and adds the predetermined ion species to water which is supplied from the external water supply source to the electrolytic water supply unit.

In the above air conditioner, it is preferable that the ion species adding unit comprises a stock tank for stocking material containing the predetermined ion species, a distributing pipe through which the stock tank and the water distributing pipe are connected to each other, an electrical conductivity detecting unit for detecting the electrical conductivity of water supplied from the water supply source, and a control unit for controlling the amount of the predetermined ion species to be added from the water supply source to the water supplied to the electrolytic water supply unit on the basis of the electrical conductivity detected by the electrical conductivity detecting unit.

In the above air conditioner, it is preferable that the control unit adds the predetermined ion species to the water when the electrical conductivity detected by the electrical conductivity detecting unit is lower than a predetermined value.

In the above air conditioner, it is preferable that the ion species adding unit further comprises an adjusting valve that is provided to the distributing pipe and adjusts the amount of the predetermined ion species to be introduced from the stock tank into the water distributing pipe, and the control unit adj usts the valve opening degree of the adjusting valve to control the amount of the predetermined ion species to be added from the water supply source to the electrolytic water supply unit.

In the above air conditioner, it is preferable that the stock tank is disposed at a low place in the room and connected to the water distributing pipe through the distributing pipe.

In the above air conditioner, it is preferable that the ion species is halide ion.

In the above air conditioner, it is preferable that the active oxygen species is at least one material selected from the group consisting of hypochlorous acid, ozone and hydrogen peroxide.

In the above air conditioner, it is preferable that the electrolytic water supply unit has a mixing unit for generating electrolytic water having a higher concentration than the concentration of electrolytic water supplied to the air filtering unit under a normal air filtering operation, and adding the higher-concentration electrolytic water concerned to water supplied from an external water supply source so that the concentration of the active oxygen species of the generated electrolytic water is equal to the prescribed concentration of the active oxygen species of the electrolytic water supplied to the air filtering unit.

In the above air conditioner, it is preferable that the electrolytic water supply unit is equipped to the housing of the indoor unit.

According to a second aspect of the present invention, there is provided an air conditioning system equipped with an outdoor unit having a compressor, a four-way valve and an outdoor heat exchanger, and one or more indoor units each having an air blower for sucking air and blowing out the air to a room and an indoor heat exchanger for heat-exchanging the air sucked by the air blower, the indoor heat exchanger and the air blower being equipped in the housing of each indoor unit, characterized in that each indoor unit has an air filtering unit for bringing the air sucked by the air blower into contact with electrolytic water containing active oxygen species sucked to filter the air, and an electrolytic water supply unit for electrolyzing water containing predetermined ion species to generate electrolytic water containing active oxygen species of a prescribed concentration is connected to the plural indoor units and supplies the generated electrolytic water to the air filtering units of the plural indoor units.

In the above air conditioning system, it is preferable that the electrolytic water supply unit generates electrolytic water having a higher concentration than the concentration of electrolytic water supplied to the air filtering unit under a normal air filtering operation, and adds the higher-concentration electrolytic water concerned to water supplied from an external water supply source so that the concentration of the active oxygen species of the generated electrolytic water is equal to the prescribed concentration of the active oxygen species of the electrolytic water supplied to the air filtering unit.

In the above air conditioning system, it is preferable that the electrolytic water supply unit has a concentration adjusting unit for controlling the amount of the higher-concentration electrolytic water to be added to the water supplied from the water supply source to thereby adjust the concentration of the active oxygen species of the electrolytic water supplied to the air filtering unit.

In the above air conditioning system, it is preferable that the electrolytic water supply unit has a concentration adjusting unit for controlling the flow rate of the water supplied from the water supply source when the higher-concentration electrolytic water is added to the water, thereby adjusting the concentration of the active oxygen species of the electrolytic water supplied to the air filtering unit.

In the above air conditioning system, it is preferable that the electrolytic water supply unit has a concentration adjusting unit for controlling the addition amount of the higher-concentration electrolytic water to the water which is supplied from the water supply source to the air filtering unit of each indoor unit, thereby adjusting the concentration of the active oxygen species of the electrolytic water supplied to each air filtering unit every air filtering unit.

In the above air conditioning system, it is preferable that the electrolytic water supply unit has a concentration adjusting unit for controlling the flow rate of the water supplied from the power supply source to the air filtering unit of each indoor unit, thereby adjusting the concentration of the active oxygen species of the electrolytic water supplied to each air filtering unit every air filtering unit.

In the above air conditioning system, it is preferable that the electrolytic water supply unit has at least a pair of electrodes, an electrolytic water bath to which water is introduced from the water supply source and in which the introduced water is electrolyzed, and an ion species adding unit for adding the predetermined ion species to the water introduced to the electrolytic bath.

In the above air conditioning system, it is preferable that the ion species adding unit comprises an electrical conductivity detecting unit for detecting the electrical conductivity of the water introduced into the electrolytic water, and a controller for controlling the amount of the ion species added to the water introduced into the electrolytic water bath on the basis of the electrical conductivity detected by the electrical conductivity detecting unit.

In the above air conditioning system, it is preferable that the ion species is halide ion.

In the above air conditioning system, it is preferable that the active oxygen species is at least one material selected from the group consisting of hypochlorous acid, ozone and hydrogen peroxide.

In the above air conditioning system, it is preferable that the housing has an air suction port through which air is sucked and an air blow-out portion through which air is blown out to the room, the air blower forms an air flowing passage in the housing so that the air sucked from the air suction port flows to the air blow-out port through the air flowing passage, and the air filtering unit is disposed on the air flowing passage to bring the air supplied through the air flowing passage into contact with the electrolytic water containing the predetermined concentration of the active oxygen species to filter the air.

It is preferable that the above air conditioning system further comprises at least one air filtering apparatus including an air blower for sucking air and blowing out the air to the room and an air filtering unit for bringing air sucked by the air blower into contact with electrolytic water containing active oxygen species to filter the air, and the air filtering apparatus is supplied with the electrolytic water from the electrolytic water supply unit.

According to a third aspect of the present invention, there is provided an air conditioning system equipped with an outdoor unit having a compressor, a four-way valve and an outdoor heat exchanger, and one or more indoor units each having an air blower for sucking air and blowing out the air to a room and an indoor heat exchanger for heat-exchanging the air sucked by the air blower, the indoor heat exchanger and the air blower being equipped in the housing of each indoor unit, characterized in that each indoor unit is equipped with an air filtering unit for bringing the air sucked by the air blower into contact with electrolytic water containing active oxygen species sucked to filter the air, and an electrolytic water supply unit for electrolyzing water containing predetermined ion species to generate electrolytic water containing active oxygen species of a prescribed concentration and supplies the generated electrolytic water to the air filtering unit, and the air conditioning system is further equipped with a water distributing pipe through which the electrolytic water supply unit of each indoor unit is connected to an external water supply source to supplywater from the external water supply source to the electrolytic water supply unit, and an ion species adding unit that is equipped in a water distributing passage formed by the water distributing pipe, adds the ion species to the water supplied from the water supply source to the electrolytic water supply unit and supplies the water added with the ion species to the electrolytic water supply units of the indoor units.

In the above air conditioning system, it is preferable that the water distributing pipe comprises a common pipe portion connected to the water supply source and distributing pipe portions that are branched from the common pipe portion and connected to the respective electrolytic water supply units of the corresponding indoor units to supply water to the indoor units, and the ion species adding unit is disposed in a water distributing passage formed by the common distributing pipe portion.

It is preferable that the above air conditioning system further comprises at least one air filtering apparatus including an air blower for sucking air and blowing out the air to the room, an air filtering unit for bringing air sucked by the air blower into contact with electrolytic water containing active oxygen species to filter the air, and an electrolytic water supply unit for electrolyzing water containing ion species to generate electrolytic water containing active oxygen species and supplies the generated electrolytic water to the air filtering unit, wherein the electrolytic water supply unit is supplied with the water added with the ion species from the ion species adding unit.

According to a fourth aspect of the present invention, there is provided an air filtering apparatus comprising: a housing having an air suction port through which air is sucked and an air blow-out port through which air is blown out to a room; an air blowing fan that is disposed in the housing and makes the air sucked from the air suction port flow to the air blow-out port; an air filtering unit that is disposed in an air flowing passage formed in the housing by the air blowing fan and brings the air supplied through the air flowing passage into contact with electrolytic water containing a predetermined concentration of active oxygen species to filter the air, and an electrolytic water supply unit for generating higher-concentration electrolytic water containing active oxygen species whose concentration is higher than the predetermined concentration, adding the higher-concentration electrolytic water with water supplied from an external water supply source so as to achieve the electrolytic water containing the active oxygen species having the predetermined concentration, and supplying the electrolytic water concerned to the air filtering unit.

According to a fifth aspect of the present invention, there is provided an air filtering apparatus comprising: a housing having an air suction port through which air is sucked and an air blow-out port through which air is blown out to a room; an air blowing fan that is disposed in the housing and makes the air sucked from the air suction port flow to the air blow-out port; an air filtering unit that is disposed in an air flowing passage formed in the housing by the air blowing fan and brings the air supplied through the air flowing passage into contact with electrolytic water containing a predetermined concentration of active oxygen species to filter the air; an electrolytic water supply unit for electrolyzing water containing a predetermined ion species to generate electrolytic water containing the active oxygen species and supplying the generated electrolytic water to the air filtering unit; and an ion species adding unit for adding the ion species to water supplied from an external water supply source to the electrolytic water supply unit.

According to a sixth aspect of the present invention, there is provided an air filtering system comprising: one or more air filtering apparatuses for filtering indoor air, each air filtering apparatus including a housing having an air suction port through which air is sucked and an air blow-out port through which air is blown out to a room, an air blowing fan that is disposed in the housing and makes the air sucked from the air suction port flow to the air blow-out port, an air filtering unit that is disposed in an air flowing passage formed in the housing by the air blowing fan and brings the air supplied through the air flowing passage into contact with electrolytic water containing a predetermined concentration of active oxygen species to filter the air, and an electrolytic water supply unit for electrolyzing water containing a predetermined ion species to generate electrolytic water containing the active oxygen species and supplying the generated electrolytic water to the air filtering unit; a water distributing pipe including a common water distributing pipe portion connected to an external water supply source and one or more distributing pipe portions that are branched from the common water distributing pipe portion and connected to the electrolytic water supply units of the respective indoor units to distribute water to the electrolytic water supply units of the respective indoor units; and an ion species adding unit that is disposed in a water distributing passage formed by the common water distributing pipe portion and adds the ion species to the water supplied from the external water supply source.

According to a seventh aspect of the present invention, there is provided an air filtering system comprising: one or more air filtering apparatuses for filtering indoor air, each air filtering apparatus comprising a housing having an air suction port through which air is sucked and an air blow-out port through which air is blown out to a room, an air blowing fan that is disposed in the housing and makes the air sucked from the air suction port flow to the air blow-out port, and an air filtering unit that is disposed in an air flowing passage formed in the housing by the air blowing fan and brings the air supplied through the air flowing passage into contact with electrolytic water containing a predetermined concentration of active oxygen species to filter the air; and an electrolytic water supply unit for generating higher-concentration electrolytic water containing active oxygen species whose concentration is higher than the predetermined concentration, adding the higher-concentration electrolytic water with water supplied from an external water supply source so as to achieve the electrolytic water containing the active oxygen species having the predetermined concentration, and supplying the electrolytic water concerned to the respective air filtering units.

According to the present invention, the air filtering unit is assembled in the indoor unit of the air conditioner. Therefore, clean air which is cooled/heated and air-filtered in a lump in the indoor unit can be blown out to the room at all times with a simple and compact construction by assembling the air filtering unit, the indoor heat exchanger and the air blower in the housing of the indoor unit.

Furthermore, according to the present invention, under the cleaning operation mode, the air filtering unit can be automatically (periodically) cleaned by supplying high-concentration electrolytic water to the air filtering unit without detaching the air filtering unit from the inside of the indoor unit and also without leakage of odor of the high-concentration electrolytic water to the room. The cleaning operation mode may be carried out while the operation of the air conditioner is stopped. Therefore, even when the operation of the air conditioner is stopped for a long term, the air filtering unit can be prevented from being dried, and thus fungus, etc. can be prevented from occurring in the air filtering unit.

Still furthermore, according to the present invention, the concentration of the electrolytic water containing the active oxygen species can be easily adjusted. Therefore, the normal air filtering operation mode and the cleaning operation mode can be easily switched to each other.

Still furthermore, according to the present invention, the scales removed from the electrolytic water supply unit can be prevented from being supplied to the air filtering unit and allowed to be discharge through the bypass flow passage by opening/closing the opening/closing valve, and also the electrolytic water generated in the electrolytic water supply unit can be prevented from being discharged through the bypass flow passage by opening/closing the opening/closing valve. The scales thus discharged may be discharged to the drain pan.

Still furthermore, according to the present invention, the electrolytic water supply unit may be supplied with water from the external water supply source. If the concentration of ion species contained in the water from the external water supply source is lower than a required concentration, the ion species adding unit adds the ion species to the water so that the water containing the ion species whose concentration is proper to the electrolysis in the electrolytic water supply unit can be supplied to the electrolytic water supply unit. This is more effective to a case where the external water supply source supplies well water, pure water, purified water or the like.

In a case where plural indoor units are provided, the ion species adding unit adds the ion species to a common pipe which is directly connected to the external water source, and also the common pipe is connected to the respective water distributing pipes which are directly connected to the electrolytic water supplyunits of the respective indoor units. Therefore, the water containing a proper concentration of ion species can be equally distributed to the respective indoor units by merely adding the ion species from the ion species adding unit to only the common pipe. Accordingly, it is unnecessary to provide the ion species adding unit every indoor unit, and thus the construction of the air conditioning system can be more simplified. In addition, the maintenance work can be also simplified.

Furthermore, according to the present invention, the electrolytic water supply unit may be designed so as to generate the high-concentration electrolytic water containing a higher concentration of active oxygen species than the electrolytic water to be supplied to the air filtering unit. The electrolytic water supply unit dilutes the higher-concentration electrolytic water with water supplied from an external water supply source to adj ust the concentration of the active oxygen species contained in the mixed electrolytic water to a predetermined concentration proper to the air filtering operation, and then supplies the adjusted electrolytic water to the air filtering unit. Therefore, in the electrolytic water supply unit, the electrolysis can be efficiently performed to generate the higher-concentration electrolytic water, and at the same time the lifetime of the electrodes can be increased.

In the case where the concentration of the active oxygen species contained in the electrolytic water supplied to the air filtering unit is adjusted to the predetermined concentration, the concentration of the active oxygen species generated simultaneously with the generation of the electrolytic water is not controlled, but the higher-concentration electrolytic water containing the higher-concentration active oxygen species in the high-concentration electrolytic water generating unit is diluted with water supplied from the water supply source to thereby adjust the concentration of the active oxygen species. Therefore, the concentration of the active oxygen species can be easily adjusted to a predetermined position by the mixing ratio between the water supplied from the water supply source and the electrolytic water.

Furthermore, the electrolytic water supply unit may be equipped separately from the indoor unit. Therefore, in a case where plural indoor units are provided, the electrolytic water can be supplied from the electrolytic water supply unit to each of the air filtering units of the indoor units, and thus it is unnecessary to provide the electrolytic water supply unit every indoor unit. Therefore, the construction of the air conditioning system can be simplified.

### BRIEF DESCRIPTION OF THE DRAWINGS

Fig. 1 is a diagram showing the construction of an air conditioner and an air conditioning system including plural indoor units according to a first embodiment of the present invention;
Fig. 2 is a cross-sectional view showing the construction of an indoor unit of a first embodiment according to the present invention;
Fig. 3 is an exploded perspective view showing the main parts of the indoor unit according to the first embodiment;
Fig. 4A is a diagram showing the construction of an air filtering unit according to the first embodiment, and Fig. 4B is a diagram showing the construction of an electrolytic water supply unit of the first embodiment;
Fig. 5 is a diagram showing a pipe arrangement in which the air filtering unit and the electrolytic water supply unit are connected to each other;
Fig. 6 is a diagram showing the construction of an air conditioner according to a second embodiment;
Fig. 7 is an exploded perspective view showing an indoor unit according to the second embodiment;
Fig. 8 is a diagram showing an air filtering unit according to the second embodiment;
Fig. 9 is a systematic diagram showing flow of electrolytic water passed through the air filtering unit;
Fig. 10 is a systematic diagram showing flow of electrolytic water passed through an air filtering unit according to a third embodiment;
Fig. 11 is a diagram showing the construction of an air conditioner and an air conditioning including plural indoor units according to a fourth embodiment;
Fig. 12 is a diagram showing the construction of the air conditioning system according to the fourth embodiment which is further equipped with an air filtering apparatus;
Fig. 13 is a diagram showing the construction of an air conditioner and an air conditioning system according to a fifth embodiment;
Fig. 14 is a cross-sectional view showing the construction of an indoor unit according to the fifth embodiment;
Fig. 15 is an exploded perspective view showing the main parts of the indoor unit according to the fifth embodiment;
Fig 16 is a diagram showing the construction of the air filtering unit according to the fifth embodiment; and
Fig. 17 is a diagram showing the construction of the air conditioning system according to the fifth embodiment.

### DETAILED DESCRIPTION OF THE PREFERRED EMBODIMENTS

Preferred embodiments according to the present invention will be described hereunder with reference to the accompanying drawings.

### (First Embodiment)

An air conditioner and an air conditioning system according to a first embodiment of the present invention will be described with reference to the accompanying drawings. In the first embodiment, a four-way air blowing and in-ceiling embedded type (or cassette type) air conditioner will be described.

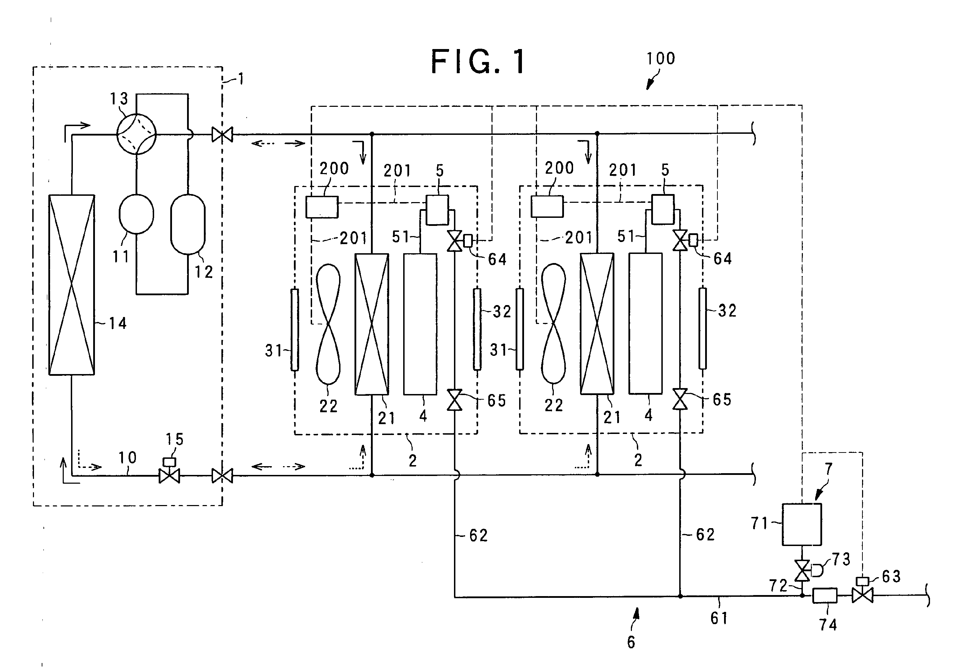

Fig. 1 is a refrigerant circuit diagram showing the construction of an air conditioner according to the first embodiment of the present invention. The air conditioner 100 shown in the refrigerant circuit diagram includes an outdoor unit 1 and two indoor units 2 connected to the outdoor unit 1. The combination of the outdoor unit 1 and the indoor units 2 is not limited to the above mode, and any combination may be adopted. For example, two or more indoor units 2 may be connected to one outdoor unit 1 or one indoor unit 2 may be connected to one outdoor unit 1. Furthermore, one or more indoor units 2 may be connected to one or more outdoor units 1.

The outdoor unit 1 is set up outdoors, and as shown in Fig. 1, it is equipped with a compressor 11, an accumulator 12, a four-way valve 13, an outdoor heat exchanger 14 and an electrically-driven expansion valve 15 which are connected to one another through a refrigerant pipe 10. In the outdoor unit 1, refrigerant flows in a direction indicated by a solid line during heating operation. Under cooling operation, refrigerant flows in a direction indicated by a broken line in Fig. 1 by switching the four-way valve 13, whereby the cooling operation and the heating operation are switched to each other.

The indoor unit 2 is set up indoors, and as shown in Fig. 1, it is equipped with an indoor heat exchanger 21, an air blowing fan 22, an air filtering unit 4, an electrolytic unit 5 (electrolytic water supply unit) and a controller 200 which are connected to each other through the refrigerant pipe 10.

The air blowing fan 22 introduces air through an air suction port 31 into the indoor unit 2, and blows out air heat-exchanged in the indoor heat exchanger 21 through an air blow-out port 32 into a room. In the air filtering unit 4, the air introduced into the indoor unit 2 by the air blowing fan 22 is brought into contact with electrolytic water containing active oxygen species such as hypochlorous acid or the like to filter air. In the electrolytic unit 5, water such as tap water or the like is electrolyzed to generate electrolytic water containing active oxygen species, and the generated electrolytic water is supplied to the air filtering unit 4. Furthermore, the controller 5 controls the current value for the electrolysis in the electrolytic unit 5.

The air filtering unit 4, the electrolytic unit 5 and the controller 200 will be described in detail later.

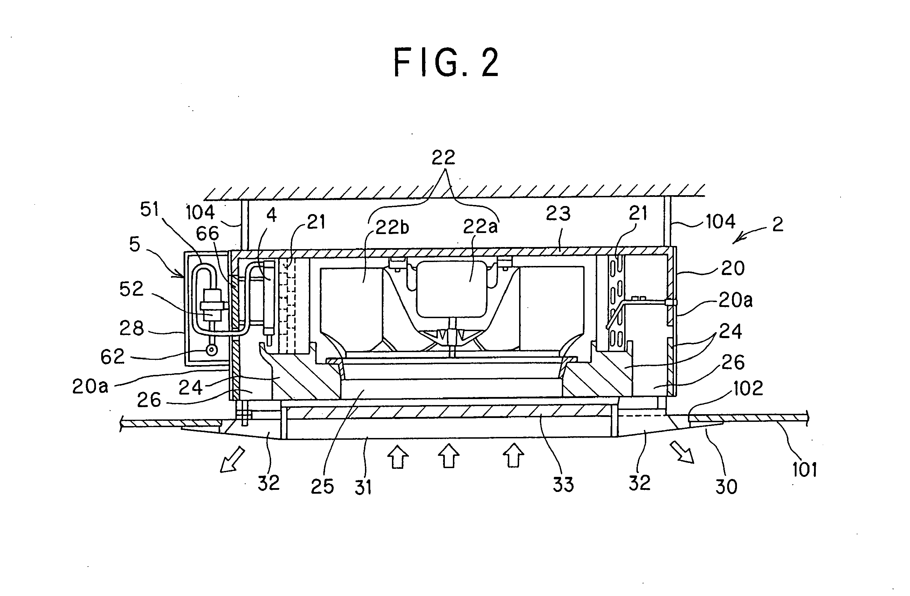

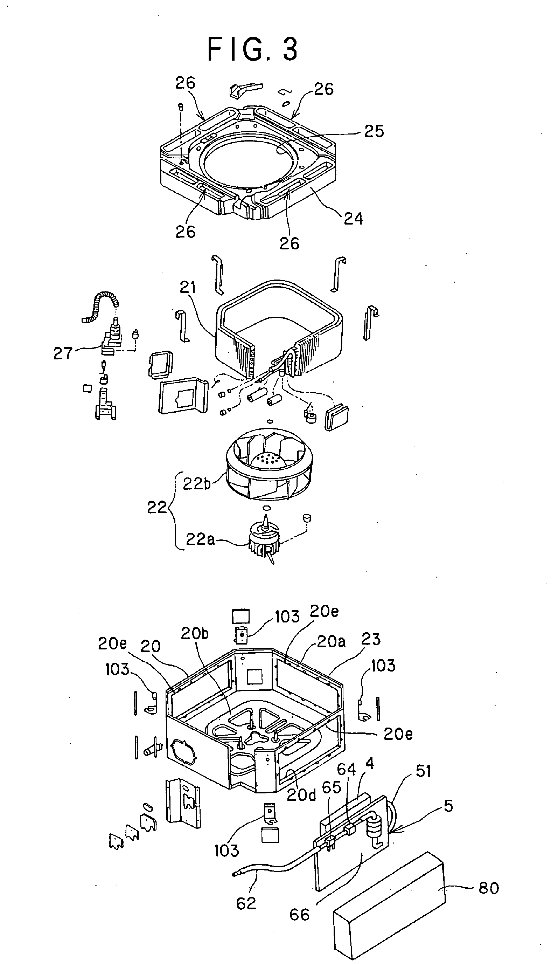

Fig. 2 is a side cross-sectional view showing the state that the indoor unit used in the air conditioner according to the first embodiment is embedded in the ceiling. Fig. 3 is an exploded perspective view showing the state that the indoor unit 2 shown in Fig. 2 is exploded while vertically turned over. In the first embodiment, two indoor units 2 are provided, and designed to have the construction.

As shown in Figs. 2 and 3, the indoor unit 2 is constructed by a housing 20 in which an indoor heat exchanger 21 and a controller 200 are accommodated, and a face panel 30 disposed at the lower side of the housing 20 under the state that the housing 20 is secured in a ceiling space S.

As shown in Figs. 2 and 3, the housing 20 is designed in a substantially rectangular box-shape having an open lower surface (at the upper side of Fig. 3). Suspending metal tags 103 are secured to the four corners of the housing 20 as shown in Fig. 1. The housing 20 is embedded from a substantially rectangular ceilinghole 102 formed in the ceiling 101 of abuilding t the back side of the ceiling 101, and the suspending metal tags 103 are fixed to suspending bolts suspended from the back side of the ceiling, whereby the housing 20 is suspended in the ceiling space S.

The face panel 30 is designed to be substantially rectangular (square) in plan view, and the lower surface of the housing 20 and the ceiling hole 102 are covered by the face panel 30. The face panel 30 is provided with an air suction port 31 located substantially at the center portion in plan view and air blow-out ports 32 elongated along the four sides of the face panel 30. A filter 33 is mounted at the back side of the air suction port 31. Accordingly, the indoor unit 2 sucks indoor air from the air suction port 31 into the housing 20, heat-exchanges the indoor air in the housing 20, and then blown out from the four air blow-out ports 32 in the four directions into the room.

As shown in Fig. 2, a thermal insulating member 23 of foamed polystyrene is provided on the inner surface of the side plate 20a of the housing 20. A motor 22a is fixed to the inside of the top plate of the housing 20, and a vane wheel 22b is secured to the shaft of the motor 22a. The motor 22a and the vane wheel 22b constitute the air blowing fan 22. An indoor heat exchanger 21 which is substantially rectangularly bent is disposed along the side plate 20a of the housing 20 so as to surround the air blowing fan 22 inside the thermal insulating member formed of foamed polystyrene (see Fig. 3). Air sucked from the air suction port 31 is supplied to the indoor heat exchanger 21 by the air blowing fan 22, and air heat-exchanged by the indoor heat exchanger 21 is blown out from the four air blow-out ports 32.

Furthermore, as shown in Fig. 2, a drain pan 24 of foamed polystyrene is disposed below the indoor heat exchanger 21. The drain pan 24 is disposed in the housing 20 under the state that the outer peripheral surface thereof is substantially provided to the inner surface of the housing 20. An air suction opening 25 and air blowing openings 26 are provided in the drain pan 24 so as to face the air suction port 31 and the air blow-out ports 32 of the face panel 30. As shown in Fig. 3, the air suction opening 25 is formed at the center of the substantially rectangular drain pan 24 so as to be substantially circular in plan view. The air blow-out openings 26 are formed along the four sides of the drain pan 24.

A drain pump 27 (see Fig. 3) is disposed at the position corresponding to one corner of the indoor heat exchanger 21 I the drain pan 24, and drain water stocked in the drain pan 21 is pumped up by the drain pump 27, and discharged to the outside of the indoor unit 2.

As shown in Figs. 2 and 3, the air filtering unit 4 is disposed at the outside of one side of the indoor heat exchanger 21 which is bent in a substantially rectangular shape. Furthermore, the electrolytic unit 5 is externally secured to a side surface of the side plate 20a of the housing 20 at which the air filtering unit 4 is disposed. The air filtering unit 4 is disposed between the side corresponding to the end portion of the indoor heat exchanger 21 and the thermal insulating member 23, and air filtered in the air filtering unit 4 is blown out from one air blow-out port 32 formed in the face panel 30. Air filtered in the air filtering unit 4 may be blown out from not only one air blow-out port 32, but also plural air blow-out ports 32.

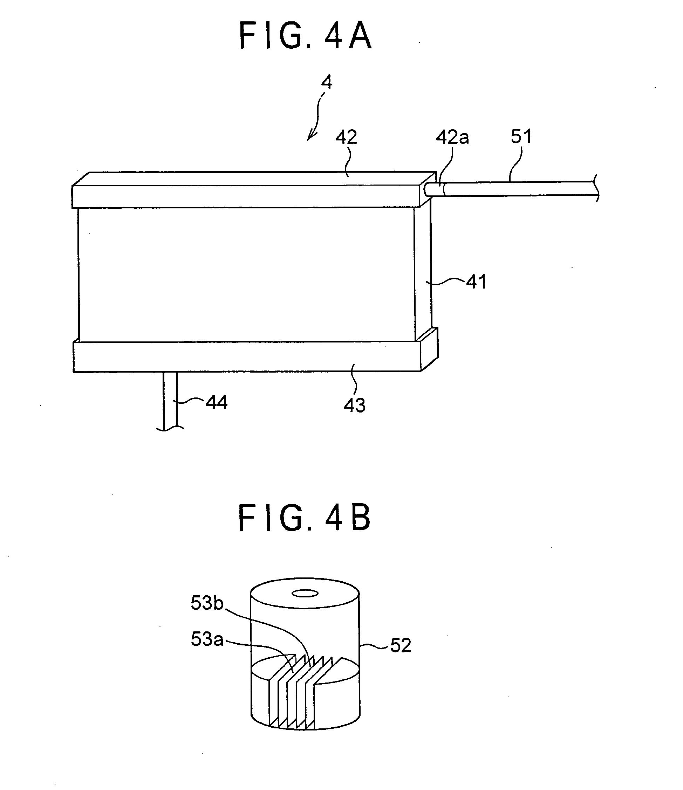

As shown in Fig. 4A, the air filtering unit 4 is equipped with a gas-liquid contact member (humidifying element) 41 having high water retentivity, a water dispersing tray 42 disposed at the upper side of the gas-liquid contact member 41 and a water receiving tray (electrolytic water tray) 43 disposed at the lower side of the gas-liquid contact member 41.

The gas-liquid contact member 41 may be constructed by non-woven cloth formed of acrylic fiber, polyester fiber or the like. A raw material having little reactivity to electrolytic water is preferably used as the raw material of the gas-liquid contact member 41, and further other materials such as polyolefin-based resin (polyethylene resin, polypropylene resin or the like), vinyl chloride resin, fluorinated rein (PTFE, PFA, ETFE or the like), cellulose-based material, ceramics-based material, etc. may be used. The gas-liquid contact member 41 maybe subjected to a hydrophilic treatment or the like to enhance the affinity of the gas-liquid contact member 41 to electrolytic water. Accordingly, the water retentivity (wettability) of the gas-liquid contact member 41 to electrolytic water is kept, and the contact between electrolytic water and introduced air can be kept for a long term.

The water dispersing tray 42 has a connection portion 42a at the right side portion in the longitudinal direction, and an electrolytic water supply pipe (electrolytic water injecting tube) 51 is connected to the connection port 42a. Many holes (hot shown) for dropping electrolytic water supplied through the electrolytic water supply pipe 51 and dispersing the electrolytic water into the gas-liquid contact member 41 are formed in the bottom surface of the water dispersing tray 42.

The water receiving tray 43 holds the gas-liquid contact member 41 from the lower side thereof, and it can stock electrolytic water passed through the gas-liquid contact member 41. A drain pipe 44 for guiding the electrolytic water to the drain pan 24 (see Figs. 2 and 3) is connected to the bottom surface of the water receiving tray 43.

The electrolytic unit 5 for supplying electrolytic water to the gas-liquid contact member 41 is disposed in an exterior box 80 as shown in Figs. 2 and 3, and the exterior box 80 is detachably secured to the side plate 20a of the housing 20. As shown in Fig. 3, the electrolytic unit 5 is provided with an electrolytic bath 52 (Fig. 4) supplied with tap water or the like from a water supply source, an opening/closing valve 64 for controlling flow of tap water or the like, and a water check valve 65 for preventing backflow of tap water or the like. The electrolytic bath 52, the opening/closing valve 64 and the water check valve 65 are intergrally secured to a fixing plate 66 as shown in Fig. 3, and the air filtering unit 4 is secured to the fixing plate 66.

As shown in Fig. 3, the air filtering unit 4 and the electrolytic unit 5 are integrally secured to the fixing plate 66, and the fixing plate 66 is secured to an opening 20d formed in the housing 20, and covered by the exterior box 80, whereby the air filtering unit 4 and the electrolytic unit 5 are assembled to the housing 20. A knock-out hole portion 20e is formed in each of three side plates 20a out of the four side plates 20a forming the outer periphery of the housing 20, and by pushing out these knock-out hole portions 20e, the opening 20d to which the air filtering unit 604, etc. are secured are formed. That is, the arrangement of the air filtering unit 4 and the electrolytic unit 5 can be properly changed in accordance with the setup position of the indoor unit 2. Furthermore, plural pairs (two or more) of the air filtering units 4 and the electrolytic units 5 may be secured to the housing 20.

At least a pair of electrodes 53a and 53b are provided in the electrolytic bath 52. When the electrodes 53a and 53b are supplied with current, tap water or the like which is supplied through a water distributing pipe 6 (see Fig. 1) to the electrolytic bath 52 and added with a predetermined ion species is electrolyzed to generate electrolytic water containing an active oxygen species.

Here, the active oxygen species means oxygen molecules having higher oxidizing activity than normal oxygen and also related substance thereof, and contain not only so-called narrowly-defined active oxygen such as superoxide anion, singlet oxygen, hydroxyl radical and hydrogen peroxide, but also so-called broadly-defined active oxygen such as ozone, hypohalous acid (hypochlorous acid, etc.), etc.

The electrolytic bath 52 is preferably disposed in proximity to the gas-liquid contact member 41. By disposing the electrolytic bath 52 in proximity to the gas-liquid contact member 41, electrolytic water containing active oxygen species achieved by electrolyzing water such as tap water or the like can be immediately supplied to the gas-liquid contact member 41.

The electrodes 52a, 52b may be constructed by two electrode plates each of which comprises a base of Ti (titan) and a coated layer of Ir (iridium), Pt (platinum).

When current is supplied to water by the electrodes 53a, 53b, the following reaction occurs at the cathode:

4H⁺ + 4e⁻ + (40H⁻) → 2H₂ + (40H⁻)

Furthermore, the following reaction occurs at the anode:

2H₂O → 4H⁺ + O₂ + 4e⁻

At the same time, chlorine ions or the like which are originally contained in tap water or the like or added by a chemical compound adder (or ion species adding unit) 7 reacts as follows:

2Cl⁻→ Cl₂ + 2e⁻

Furthermore, Cl₂ thus generated reacts with water as follows:

Cl₂ + H₂O → HClO + HCl

In this construction, by supplying current to the electrodes 53a, 53b, HClO (hypochlorous acid) having strong sterilizing power, etc. are generated. Therefore, air is passed through the gas-liquid contact member 41 supplied with the electrolytic (filtering) water containing this hypochlorous acid or the like to inactivate or sterilize virus, etc. floated in the airpassing through the gas-liquid contact member 41, thereby filtering the air, and also breeding of various bacteria, fungus, etc. in the gas-liquid contact member 41 can be prevented. Furthermore, when gaseous materials such as odor components, etc. pass through the gas-liquid contact member 41, the odor components are dissolved in the electrolytic water or also react with hypochlorous acid or the like in the filtering water, whereby the odor components can be removed from air and thus the air is deodorized.

When electrolytic water is generated, the concentration of the active oxygen species in the electrolytic water is adjusted to such a value that the virus, etc. to be filtered can be inactivated. The adjustment of the concentration of the active oxygen species can be performed by adjusting the voltage applied between the electrodes 53a, 53b and thus adjusting the current value flowing between the electrodes 53a, 53b.

For example, when a desired amount of current (20mA/cm² in current density) is supplied between the electrodes 53a, 53b, a prescribed amount of tap water or the like is electrolyzed to generate a predetermined concentration of active oxygen species (for example, a predetermined free residual chlorine concentration of 1mg/liter can be generated. By changing the current value, for example, by reducing the current value, the concentration of hypochlorous acid of the electrolytic water can be reduced. By increasing the current value, the concentration of hypochlorous acid of the electrolytic water can be increased. The change of the current value may be performed by changing the current value between the electrodes 53a and 53b with a variable resistor (not shown). The resistance value of the variable resistor is determined on the basis of a signal from the controller 200.

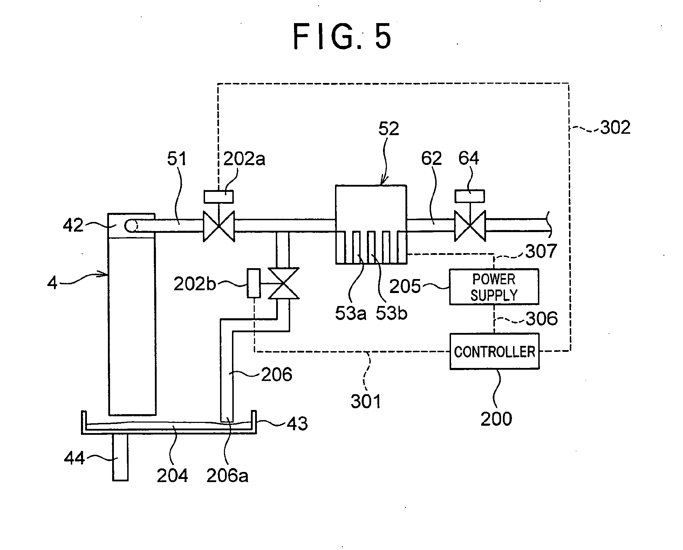

As shown in Fig. 5, the electrolytic unit 5 is connected to a water supply source (not shown) through a water distributing pipe 6 and also connected to the air filtering unit 4 through the electrolytic water supply pipe 5.

The water supply source is not limited to a specific one insofar it can supply water to the electrolytic unit 5 through the water distributing pipe 6. For example, the water distributing pipe 6 may be connected to a water pipe (not shown) so that city water (tap water) supplied through the water pipe 6 is used as the water supply source, or the water distributing pipe 6 may be connected to a water supply bath (not shown) or the like so that water stocked in the water supply bath or the like is used as the water supply source. Here, the water stocked in the water supply bath or the like may be water which contains ion species such as chloride ions or the like in advance, or water having a low concentration of ion species such as well water or the like. The water to be supplied from the water supply source through the water distributing pipe 6 will be referred to as "tap water or the like".

The water distributing pipe 6 is equipped with a water supply source connecting pipe (common water pipe or water supply source connecting unit) 61 to be connected to the water supply source, and a water distributing pipe 62 (water distributing unit) which is branched from the water supply source connecting pipe 61 to each indoor unit 2 to distribute tap water or the like to the electrolytic unit 5. The water supply source connecting pipe 61 and the water distributing pipe 62 are provided with opening/closing valves 63 and 64, respectively, and the opening/closing valves 63 and 64 are opened to supply tap water or the like to the electrolytic unit 5.

From the aspect of maintenance, a stock tank 71 may be disposed as the water supply source in the neighborhood of the floor (at a low place), for example, the stock tank 71 may be mounted on the floor. In this case, the stock tank 71 may be connected to the water supply source connecting pipe 61 disposed under floor or the like through the distributing pipe 72. The stock tank 71 is located not at a high place, but at a low place (i.e., in the neighborhood of the floor), thereby facilitating supply of chemical material or the like into the stock tank 71.

The water distributing pipe 6 is provided with a chemical material adder 7 for adding chemical material containing a predetermined ion species to tap water or the like which is supplied from the water supply source through the water supply source connecting pipe 61. The chemical material adder 7 is equipped with a stock tank 71 for stocking chemical material containing a predetermined ion species, a distributing pipe 72 for joining the stock tank 71 and the water supply source connecting pipe 61, a flow rate adjusting valve 73 for detecting the electrical conductivity of tap water or the like supplied from the water supply source, an electrical conductivity meter 74 for detecting the electrical conductivity of tap water or the like supplied from the water supply source, and a controller (not shown) for adjusting the opening degree of the flow rate adjusting valve 73 on the basis of the electrical conductivity of tap water or the like detected by the electric conductivity meter 74 to control the amount of the chemical material to be added to tap water or the like.

Here, the predetermined ion species is preferably halide ions (X⁻), andparticularlychlorideions (Cl⁻) aremorepreferably. When chloride ions are added as the predetermined ion species to tap water or the like, natrium chloride (NaCl) or potassium chloride (KCL) may be used as a material containing chloride ions (electrolyte). With respect to natrium chloride, salt or the like may be added to tap water or the like while it is under a solid state, or it may be added to tap water or the like while salt is adjusted in water solution like brine. In this embodiment, brine whose concentration is adjusted to a predetermined concentration is stocked in the stock tank 71. By adding a predetermined ion species to tap water or the like under a water-solution state like brine, the predetermined ion species can be safely and easily added to tap water or the like.

The electrical conductivity meter 74 detects the electrical conductivity of tap water or the like flowing from the water supply source into the water distributing pipe 6.

Here, the concentration of the ion species in tap water or the like can be estimated by detecting the electrical conductivity of tap water or the like. That is, even when the same amount of tap water or the like is supplied to the electrolytic unit 5 and current having the same current density is made to flow between the electrodes, the concentration of the active oxygen species such as hypochlorous acid or the like generated in the electrolytic unit 5 is varied in accordance with the concentration of chloride ions or the like in the tap water or the like. Therefore, it is difficult in some cases to generate electrolytic water containing an active oxygen species efficiently in accordance with the concentration of ion species contained in tap water or the like. Therefore, the concentration of the ion species such as chloride ions or the like is estimated on the basis of the electrical conductivity of tap water or the like supplied from the water supply source, and a predetermined ion species is added in accordance with the estimated concentration. At this time, when the concentration of the ion species such as tap water or the like supplied from the water supply source is less than a predetermined concentration (for example, 30ppm or less in chloride ions or the like), the valve opening degree of the flow rate adjusting valve 73 may be adjusted so that a predetermined amount of ion species is added, or the valve opening degree of the flow rate adjusting valve 73 may be adjusted in accordance with variation of the electrical conductivity so that the concentration of the ion species in tap water or the like supplied to the electrolytic unit 5 is equal to a predetermined value.

In the water supply source connecting unit, a predetermined ion species is added and tap water or the like which contains a predetermined concentration or more of the ion species is supplied to the electrolytic unit 5 of each indoor unit 2 by the distributing pipe 62 of the water distributing pipe 6.

A predetermined ion species is added to tap water or the like supplied to the electrolytic unit 5, and thus it is preferable that the water distributing pipe 6 is formed of a material that has resistance to tap water or the like containing a predetermined ion species and does not induce deterioration of the water distributing pipe 6 such as rust, etc. A plastic pipe or a composite pipe whose inside is formed of iron and coated with plastic material may be used for the water distributing pipe 6. For example, a chloride vinyl pipe, a polyethylene pipe, a polybutene pipe, a cross-linked polyethylene pipe or the like may be used as the plastic pipe. Furthermore, a resin lining steel pipe or the like may be used as the composite pipe.

Furthermore, as shown in Fig. 1, the controller 200 provided to the indoor unit 2 is connected to the air blowing fan 22, the electrolytic unit 5 and the opening/closing valves 63 and 64 through wires 201. The controller 200 can control start/stop the operation of the air blowing fan 22, and also can variably control the current value of the electrolysis in the electrolytic unit 5. In more detail, the air conditioner 100 has a cleaning operation mode for cleaning the air filtering unit 4 by carrying out the above control operation.

The cleaning operation mode is as follows. During the normal operation of the indoor unit 2, electrolytic water containing an active oxygen species of about 2ppm in concentration is supplied to the air filtering unit 4. However, under a predetermined condition, electrolytic water containing an active oxygen species of a higher concentration (for example, 10ppm or more) than the concentration of the electrolytic water under the normal operation of the air filtering unit 4 is supplied to the air filtering unit 4 to clean the air filtering unit 4.

The following operation control is carried out in the cleaning operation mode.
(1) When the operation of the indoor unit 2 is stopped
   When the operation of the indoor unit 2 is continuously stopped for three hours or more, electrolytic water has not been supplied to the air filtering unit 4. Therefore, there is a risk that the air filtering unit 4 is dried, so that fungus or the like occurs in the air filtering unit 4 or bacteria breed. In order to prevent the above risk and also effectively clean the air filtering nit 4, it is necessary to supply the air filtering unit 4 with electrolytic water of a high concentration.
   In this case, the controller 200 detects that the operation of the indoor unit 2 is stopped, opens the opening/closing valves 63, 64 every predetermined time (for example, three hours), set the current value for the electrolysis to a high value in the electrolytic unit 5 to generate electrolytic water of high concentration, and supplies the generated electrolytic water to the air filtering unit 4. Accordingly, the air filtering unit 4 can clean the air filtering unit 4 every predetermined time (every three hours). The time interval (three hours) is an example, and thus it may be set to another proper time interval.
(2) When the indoor unit 2 is under continuous operation
   When operation of the indoor unit 2 is continuously operated for 24 hours, for example, the air filtering unit 4 may be much stained. Therefore, it is necessary to periodically supply electrolytic water of high concentration to the air filtering unit 4.

In this case, the controller 200 detects that the operation is continued for 24 hours, and it stops the air blowing fan 22 for only a predetermined time in a time zone in the middle of the night, for example, Then, the controller 200 controls the air filtering unit so that the current value for the electrolysis in the electrolytic unit 5 is set to a high value, electrolytic water of high concentration is generated and the generated electrolytic water is supplied to the air filtering unit 4. accordingly, during the cleaning operation mode, the air filtering unit 4 can be cleaned in a predetermined time zone.

The controller 200 may also serve as the controller for controlling the switching of the four-way valve 13, the opening degree of the flow rate adjusting valve 73, etc. to control the air conditioner 100.

Fig. 5 is a diagram showing a pipe passage for connecting the air filtering unit 4 and the electrolytic unit 5.

The electrolytic water supply pipe 51 through which the air filtering unit 4 is connected to the electrolytic bath 52 is provided with a bypass pipe (bypass flow path) 206 which is branched from the water flow passage of the electrolytic water supply pipe 51 at some position between the electrolytic unit 52 and the air filtering unit 4. The discharge portion 206a of the bypass pipe 206 extends to the water receiving tray 43 of the air filtering unit 4, so that electrolytic water sent from the electrolytic unit 5 and foreign materials such as scales or the like described later can be discharged through the bypass pipe to the water receiving tray 43. The bypass pipe may be connected to the drain pan disposedbelow the indoor heat exchanger, or it may be connected to at least one of the water receiving tray 43 and the drain pan.

Furthermore, the electrolytic water supply pipe 51 is also provided with an opening/closing valve 202a for adjusting the flow rate of electrolytic water which is located at the downstream side of the branch position with respect to the flow direction of the electrolytic water from the electrolytic bath 52, and the bypass pipe 206 is also provided with an opening/closing valve 202b for adjusting the flow rate of electrolytic water supplied to the water receiving tray 43. These opening/closing valves 202a and 202b are connected to the controller 200 through signal lines 301 and 302, and the flow rate of the electrolytic water is adjusted on the basis of a signal from the controller 200.

The controller 200 is connected through signal line 306 to a power supply device 205 for applying a voltage to the electrodes 53a and 53b of the electrolytic bath 52. The power supply device 205 is electrically connected to the electrodes 53a and 53b of the electrolytic bath 52 through a wire 307, and can invert the polarities (plus and minus polarities) of the electrodes 53a and 53b on the basis of the signal from the controller 200.

When the voltage is applied to the electrodes 53a and 53b of the electrolytic bath 52 in the above construction, scales 204 adhere to the electrodes 53a, 53b due to calcium ions or magnesium ions contained in electrolytic water used to generate electrolysis. Therefore, it is required to periodically remove the scales 204 from the electrodes 53a and 53b and thus prevent the electrolysis performance and durability of the electrolytic bath 52 frombeing lowered. The removable of the scales is carried out according to the following procedure.
(1) Control of Opening/closing Valves 202a and 202b
   During normal operation (when electrolytic water is supplied), the opening/closing valve 202a is opened, and the opening/closing valve 202b is closed, whereby the electrolytic water generated in the electrolytic bath 52 is supplied from the electrolytic water supply pipe 51 to the air filtering unit 4, and it is prevented from flowing to the bypass pipe 206. On the other hand, in order to remove scales 204, the opening/closing valve 202a is closed and the opening/closing valve 202b is opened, whereby the removed scales 204 are passed through the bypass pipe 206 without flowing to the electrolytic water supply pipe 51, and then discharged to the water receiving tray 43.
(2) Inversion of Polarities of Electrodes 53a, 53b

On the basis of the signal from the controller 200, the polarities of the electrodes 53a, 53b under electrolysis operation are inverted. This is because the scales 204 are formed on the electrode at the cathode side, and the probability that the scales exfoliate from the electrode is increased by inverting the polarities of the electrodes. Accordingly, the removed scales 204 are passed through the bypass pipe 206 and then discharged to the water receiving tray 43.

Furthermore, according to the air conditioner of the first embodiment, the concentration of active oxygen species such as hypochlorous acid or the like in electrolytic water generated through electrolysis can be freely adjusted by controlling current applied to the electrodes 53a, 53b when tap water or the like is electrolyzed in the electrolytic unit 5. Accordingly, electrolytic water of higher concentration (i.e., electrolytic water containing active oxygen species of higher concentration) than electrolytic water supplied for air filtering (i.e., under the normal air filtering operation) can be supplied to the air filtering unit 4, and thus the air filtering unit 4 can be cleaned by the higher-concentration electrolytic water. Accordingly, the air filtering unit 4 can be cleaned without detaching the air filtering unit 4 itself from the inside of the indoor unit 2, and thus the cleaning work can be easily performed.

By executing the cleaning operation mode for cleaning the air filtering unit 4 under the control of the controller 200, the air cleaning unit 4 can be periodically (automatically) cleaned without any user's labor.

Furthermore, by stopping the operation of the air blowing fan when the air filtering unit 4 is cleaned, odor emitted from the electrolytic water of high concentration can be prevented from being actively diffused to the room side.

In the cleaning operation mode, the electrolytic water of high concentration is supplied to the air filtering unit 4 at a predetermined time interval (for example, every three hours) while the operation of the indoor unit 2 is stopped. Therefore, the air filtering unit 4 can be prevented from being dried and also bacteria, fungus, etc. can be prevented from breeding in the air filtering unit 4. Accordingly, the inside of the indoor unit 2 can be kept clean.

Furthermore, in the cleaning operation mode, electrolytic water of high concentration is supplied in a midnight time zone under which there is little user when the operation of the indoor unit 2 is continuously carried out. Therefore, the air filtering unit 4 can be periodically cleaned even in the air conditioner which is continuously operated over 24 hours. Accordingly, the inside of the indoor unit 2 can be kept clean.

According to the first embodiment, the air filtering unit 4 is provided to the air blow-out port 32 side of the indoor unit 2, and thus the active oxygen species such as hypochlorous acid or the like can be prevented from being directly introduced to the indoor heat exchanger 21. Therefore, the indoor heat exchanger 21 can be prevented from being corroded by the active oxygen species such as hypochlorous acid or the like.

According to the air conditioner of the first embodiment of the present invention, the bypass pipe 206 branched from some midpoint of the electrolytic water supply pipe 51 is provided. Therefore, the scales 204 occurring in the electrolytic unit (electrolytic bath 52) can be made to flow through the bypass pipe 206. Accordingly, the scales can be prevented from being supplied to the air filtering unit 4 and thus the air filtering unit 4 can be prevented from being stained by the scales 204.

Furthermore, the opening/closing valves 202a and 202b are provided, and the opening/closing operation of the opening/closing valves is carried out under the normal operation and under the scale removing operation, whereby the removed scales 204 canbe surelymade to flow to the bypass pipe 206. Accordingly, no scale is supplied to the air filtering unit 4 and thus the air filtering unit 4 can be prevented from being stained by the scales 204.

Still furthermore, the discharging portion 206a of the bypass pipe 206 is provided so as to extend to the water receiving tray 43, so that the discharged scales can be discharged together with the electrolytic water which has passed through the air filtering unit. Accordingly, the device, etc. for discharging the removed scales can be commonly used, so that the number of parts of the air conditioner 100 can be reduced and the cost can be also reduced.

The control of the opening/closing valves 202a, 202b and the inversion of the polarities of the electrodes 53a, 53b are carried out on the basis of the control signals of the controller 200. Therefore, the operation mode such as the scale removing operation for periodically removing scales can be automatically carried out. As a result, there can be provided the air conditioner 100 that can be more efficiently operated.

Various modifications and alterations may be made to the first embodiment without departing from the subject matter of the present invention.

In the first embodiment, in order to increase the concentration of electrolytic water, the current value for electrolysis is controlled by the controller 200. However, the opening/closing valves 63, 64 may be constructed by the flow rate adjusting valve so that the concentration of electrolytic water to be achieved through the electrolysis can be changed by adjusting/changing the flow rate of tap water or the like supplied to the electrolytic unit 5. The concentration of electrolytic water can be changed by controlling the flow rate with the flow rate adjusting valve 73 and adjusting/changing the amount of chemical material to be added to the tap water or the like by the chemical material adder 7. Accordingly, the air filtering unit can be cleaned by electrolytic water of high concentration as in the case of the above embodiment.

Furthermore, in the first embodiment, the description has been made by exemplifying a so-called in-ceiling embedded type four-way blow-out type indoor unit 2. However, the present invention is not limited to this type indoor unit, and the indoor unit may be a ceiling-suspended type, a wall-suspended type or an on-floor mount type. Furthermore, the air filtering unit 4 is disposed between the thermal insulating member 23 and one side of the indoor heat exchanger 21 which is disposed in the housing 20 of the indoor unit 2 and bent in a substantially rectangular shape. However, it is needless to say that the air filtering unit 4 may be disposed one or more sides. Furthermore, the gas-liquid contact member 41 of the air filtering unit 4 may be disposed substantially in parallel to or obliquely to the indoor heat exchanger 21.

In the first embodiment, the bypass pipe 206 is provided so as to be branched from the electrolytic water supply pipe 51, however, the bypass pipe 206 may be provided at any position and in any style insofar as it is located at the upstream side of the air filtering unit 4. For example, the electrolytic water supply pipe 51 and the bypass pipe 206 may be provided so as to individually extend from the electrolytic unit 5 (electrolytic bath 52) so that the removed scales are discharged from the bypass pipe 206.

Furthermore, the discharging portion 206a of the bypass pipe 206 is extended to the water receiving tray 43 of the air filtering unit 4 to discharge the scales 204 to the water receiving tray 43. However, the discharging portion 206a may be extended to the drain pan 24 at the lower side of the indoor heat exchanger 21 to discharge the scales 204 to the drain pan 24.

### (Second Embodiment)

An air conditioner according to a second embodiment of the present invention will be described hereunder.

The second embodiment of the present invention will be also described by exemplifying an four-way blow-out type in-ceiling embedded air conditioner.

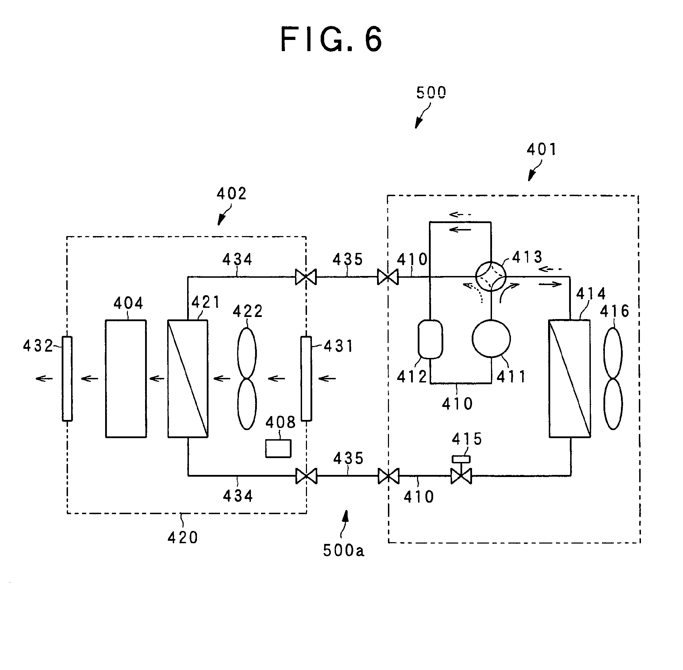

Fig. 6 shows the construction of an air conditioner 500 according to the second embodiment. The air conditioner 500 of the second embodiment is a separation type heat pump type air conditioner having an outdoor unit 401 and an indoor unit 402. An outdoor refrigerant pipe 410 of the outdoor unit 401 and an indoor refrigerant pipe 434 of the indoor unit 402 are connected to each other through a joint pipe 435 and the operation of each of the outdoor unit 401 and the indoor unit 402 is controlled by the controller 408.

The outdoor unit 401 is set up outdoors. As shown in Fig. 6, a compressor 411 is disposed in the outdoor refrigerant pipe 410, an accumulator 412 is connected to the suction side of the compressor 411, and a four-way valve 413, an outdoor heat exchanger 414 and an electrically-driven expansion valve 415 are successively connected to the discharge side of the compressor 411 in this order. An outdoor fan 416 for blowing air to the outdoor heat exchanger 414 is disposed in the outdoor unit 401.

The indoor unit 402 is set up in a room to be air-conditioned. As shown in Fig. 6, the indoor unit 402 has a housing 420 having an air suction port 432 to an air blow-out port 432, an indoor heat exchanger 421, an air blowing fan (air blower) 422 for blowing air from the air suction port 431 to the air blow-out port 432 in the housing 420, and an air filtering unit 404 that is disposed in an air flowing passage formed by the air blowing fan 422 in the housing 420 and brings air heat-exchanged in the indoor heat exchanger 421 into contact with electrolytic water containing active oxygen species to filter the air. The indoor heat exchanger 421, the air blowing fan 422 and the air filtering unit 404 are accommodated in the housing 420.

The controller 408 has CPU, ROM and RAM (not shown). CPU controls the overall air conditioner 500 according to a control program in ROM. ROM stores control data containing the control program in advance. RAM temporarily stores various kinds of data.

In the air conditioner 500, by switching the four-way valve 413, the flow of refrigerant flowing in the refrigerant circuit 500a is switched, thereby switching the cooling operation and the heating operation to each other. Under cooling operation, the refrigerant flows in a direction indicated by a solid-line arrow in Fig. 6, and under heating operation, the refrigerant flows in a direction indicated by a broken-line arrow in Fig. 6.

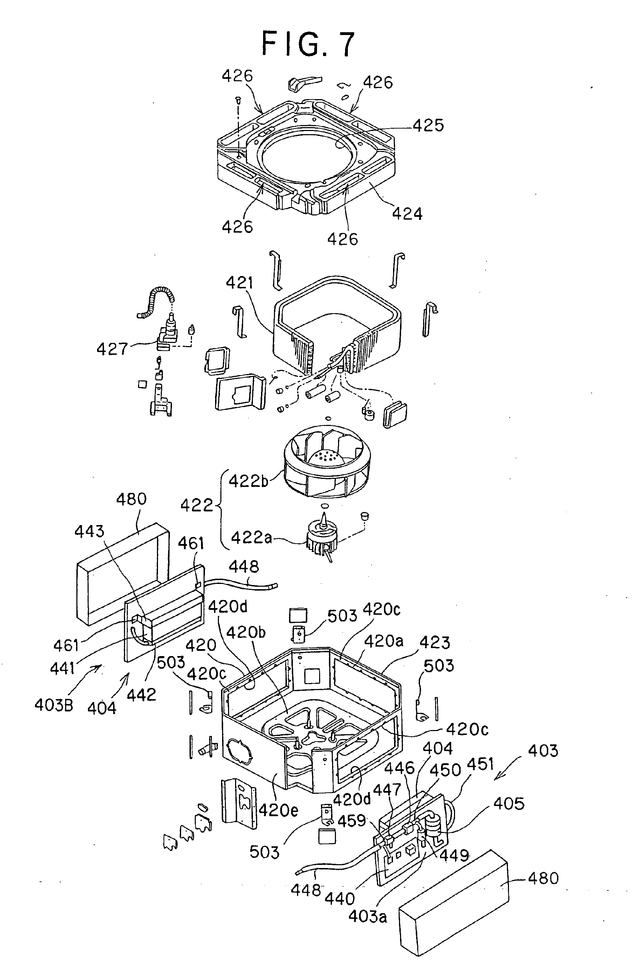

Fig. 7 is an exploded perspective view showing the state that the indoor unit 402 is exploded while vertically turned over, and corresponds to Fig. 3. The embedded state of the indoor unit 402 is the same as that shown in Fig. 2.

In the second embodiment, as in the case of Fig. 2, the indoor unit 402 includes the housing 420 in which the indoor heat exchanger 421, the controller 408, etc. are accommodated, and a face panel 430 disposed at the lower side of the housing 420.

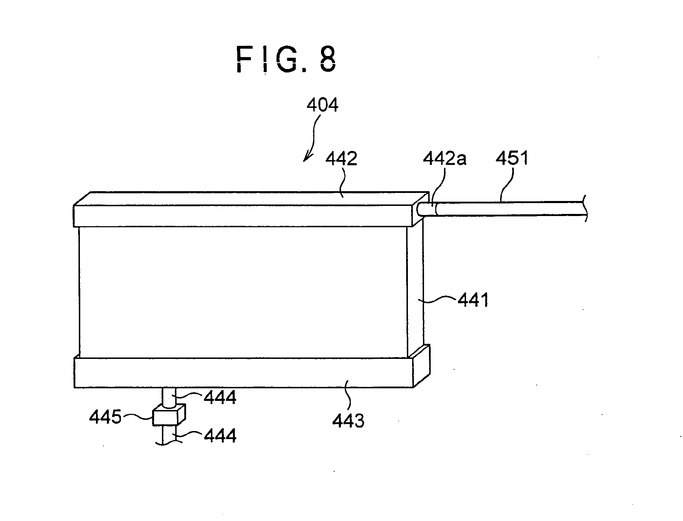

As shown in Fig. 7, the housing 420 is designed in a substantially rectangular box-like shape having an open lower surface (upper surface in Fig. 8). The housing 420 has a side plate 420 having a cut-out for guiding an indoor refrigerant pipe 434, etc. to be connected to the indoor heat exchanger 421, and three side plates 420a each of which is provided with a knock-out hole portion 420c at which an opening is formed by pushing something against the side plate 420c. A filtering unit 403 to which an air filtering unit 404, etc. are secured is put into an opening 420d formed at the knock-out hole portion 420c from the outside. Here, when the opening 420d is formed in only one knock-out hole portion 420c, only one filtering unit 403 is secured to the indoor unit 402. However, when the openings 420d are formed in two or more knock-out holes 420, two or more filtering units 403 can be secured to the indoor unit 402. In Fig. 7, the filtering units 403a and 403b are secured to the indoor unit 402.

The filtering unit 403 has a plate portion 403a which is put in the opening 420d and closes the opening 420d, and the air filtering unit 404 is secured to the inside of the plate portion 403a through a fixing tag 461. Since the air filtering unit 404 is secured to the plate portion 403a through the fixing tag 461, a gap is formed between the plate portion 403a and the air filtering unit 404. A thermal insulating member of foamed polystyrene is provided on the inner side surface of the plate portion 403a.

An electrolytic unit (electrolyzing unit) described in detail later, a water (tap water or the like) control valve 446, a check valve 447, a circulating pump 449, an electrical component board (controller) 440, etc. are secured to the outside of the plate portion 403a. The filtering unit 403 is covered by an exterior cover 480.

Here, the electrical component board 440 is equipped with CPU, ROM and RAM, and connected to the controller 408. CPU controls the electrolytic unit 405, the water control valve 446, a circulating pump 449, etc. according to the control program in ROM. Accordingly, on the basis of an instruction of the controller 408, the electrical component board 440 adjusts the flow rate of water such as tap water or the like by opening/closing the water control valve 446 generates electrolytic water by supplying current to the electrolytic unit 405, drives the circulating pump 449 to circulate the electrolytic water, etc. ROM stores control data containing the control program in advance. RAM temporarily stores various kinds of data.

Furthermore, as shown in Fig. 7, suspending metal tags 503 are secured to the four corners of the housing 420. As described above with respect to Fig. 2, the housing 420 is embedded from a substantially rectangular ceiling hole formed in the ceiling of a building to the back side of the ceiling, and the suspending metal tags 503 are fixed to suspending bolts suspended from the back side of the ceiling, whereby the housing 20 is suspended in the ceiling space S.

The internal construction of the housing 420 of this embodiment is basically the same as that of Fig. 2, and thus the detailed description thereof is omitted from the following description. Only the difference of the second embodiment from the first embodiment will be mainly described below.

The filtering unit 403 is disposed at one portion of the side surface of the substantially rectangularly bent indoor heat exchanger 421 so that the air filtering unit 404 is adjacent to the outside of the indoor heat exchanger 421. The air filtering unit 404 is disposed between the side surface of the indoor heat exchanger 421 and the plate portion 403a of the filtering unit 403a having the thermal insulating member. Air passed through the indoor heat exchanger 421 is filtered in the air filtering unit 404. The filtered air flows downwardly between the air filtering unit 404 and the plate portion 403a, and blown out from the air blow-out ports 432 formed in the face panel 430 to the room to be air-conditioned.

As shown in Fig. 8, the air filtering unit 404 is equipped with a gas-liquid contact member 4 41 having high water retentivity, a water dispersing tray 442 disposed at the upper side of the gas-liquid contact member 441 and a water receiving tray 443 disposed at the lower side of the gas-liquid contact member 441.

The material constituting the gas-liquid contact member 441 of the second embodiment is the same as that of the first embodiment. That is, the gas-liquid contact member 441 may be constructed by non-woven cloth formed of acrylic fiber, polyester fiber or the like. A raw material having little reactivity to electrolytic water is preferably used as the raw material of the gas-liquid contact member 441, and further other materials such as polyolefin-based resin (polyethylene resin, polypropylene resin or the like), vinyl chloride resin, fluorinated rein (PTFE, PFA, ETFE or the like), cellulose-based material, ceramics-based material, etc. may be used. Furthermore, the gas-liquid contact member 441 may be subjected to a hydrophilic treatment or the like to enhance the affinity of the gas-liquid contact member 441 to electrolytic water. Accordingly, the water retentivity (wettability) of the gas-liquid contact member 41 to electrolytic water is kept, and the contact between electrolytic water and introduced air can be kept for a long term.

The water dispersing tray 42 has a connection portion 442a connected to the electrolytic water supply pipe 451 in the side surface thereof the right side portion. Many holes (hot shown) for dropping electrolytic water supplied through the electrolytic water supply pipe 451 and dispersing the electrolytic water into the gas-liquid contact member 441 are formed in the bottom surface of the water dispersing tray 442.

The water receiving tray 443 can stock electrolytic water passed through and dropped from the gas-liquid contact member 41. A drain pipe 444 for guiding the electrolytic water to the drain pan 424 (Fig. 7) is connected to the bottom surface of the water receiving tray 443. Both the ends of the water receiving tray 443 are supported by fixing tags 461 to secure the air filtering unit 404 to the plate portion 403a.

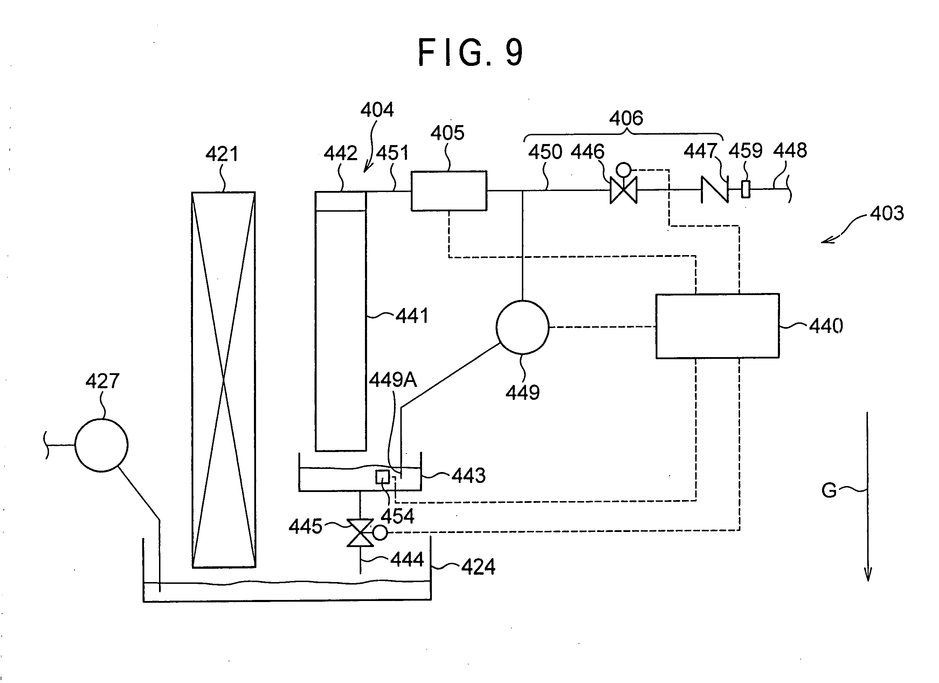

The electrolytic water supply pipe 451 is connected to a water supply source (not shown) through the electrolytic unit 405 and a water (tap water or the like) adjusting unit (water supply unit) 406 (see Fig. 9). The water supply source is not limited to a specific one insofar as it supplies through the water adjusting unit 406 to the electrolytic unit 405. For example, the water adjusting unit 406 is connected to a water pipe (not shown) and city water (tap water) to be supplied through the water pipe may be used as the water supply source. Furthermore, the water adjusting unit 406 may be connected to a water supply bath (not shown) or the like to use water stocked in the water supply bath or the like as the water supply source. Here, water stocked in the water supplybath or the likemaybe water containing ion species such as chloride ions or the like in advance (for example, tap water or the like), or water containing ion species of low concentration such as well water or the like.

The construction of the electrolytic unit 405 for generating electrolytic water and supplying the generated electrolytic water to the gas-liquid contact member 441 is basically the same as that of Fig. 4B, and thus the detailed description thereof is omitted from the following description. Furthermore, the active oxygen species generated by electrolyzing tap water or the like and the chemical reaction process of generating electrolytic water containing the active oxygen species are the same as described with reference to the first embodiment, and thus the detailed description thereof is omitted from the following description.

Fig. 9 is a diagram showing the flow of electrolytic water passed through filtering unit 404. In Fig. 9, the direction indicated by an arrow G is set to the downward direction.

As described above, the water adjusting unit 406 is connected to the upstream side of the water adjusting unit 405 as described above, and the water adjusting unit 406 is connected to the water supply source through a water (tap water or the like) injecting pipe 448.

The water adjusting unit 406 is equipped with a connecting pipe 450, a water control valve (water supply valve) 446 and a check valve 447. The electrolytic unit 405 is connected to the water control valve 446 through the connection pipe 450, and the water control valve 446 is connected to the check valve 447. The water injection pipe 448 is connected to the check valve 447 through a connector 459.

The water control valve 446 carries out the opening/closing operation on the basis of the control of the electrical component board 440, and tap water or the like supplied from the water supply source flows into the electrolytic unit 45 when it is opened.

The electrolytic water supplied from the electrolytic unit 405 to the air filtering unit 4 is stocked in the water receiving tray 443. A suction nozzle 449A of a circulating pump 449 is disposed in the water receiving tray 443, and thus the water receiving tray 443 is connected to the connection pipe 450 through the circulating pump 449. Accordingly, the electrolytic water stocked in the water receiving tray 443 is supplied to the electrolytic unit 405 by driving the circulating pump 449. The drain pipe 444 connected to the lower portion of the water receiving tray 443 is equipped the flow amount (rate) control valve 445 as an electromagnetic valve. Accordingly, when the flow amount (rate) control valve 445 is opened, the electrolytic water stocked in the water receiving tray 443 flows to the drain pipe 444. The drain pipe 444 is disposed above the drain pan 424, and the electrolytic water flowing through the drain pipe 444 flows from the lower end of the drain pipe 444 to the drain pan 424.

The water receiving tray 443 is provided with a float switch (water level detecting unit) 454 for detecting the water level of the electrolytic water stocked in the water receiving tray 443. The float switch 454 is connected to the electrical component board 440. The electrical component board 440 opens the water control valve 446 to supply tap water or the like to the electrolytic unit 405 when the float switch 454 detects that the water level is low (not more than the water level required to circulate electrolytic water).

Next, the operation of the air conditioner 500 according to the second embodiment will be described.

When an operation start instruction is input from an indoor remote controller (not shown) or the like by a user, the controller 408 switches the four-way valve 413 of the outdoor unit 401 to a cooling side or heating side in accordance with the operation mode (cooling operation mode/heating operation mode) indicated by the operation start instruction to carry out a predetermined air-conditioning operation such as a cooling operation, a heating operation or the like.

When the cooling operation is carried out, the controller 408 switches the four-way valve 413 to the cooling side so that refrigerant flows into the refrigerant circuit 500a, the outdoor heat exchanger 414 served as a condenser and the indoor heat exchanger 421 serves as an evaporator as indicated by a broken-line arrow of Fig. 6. Then, the air blowing fan 422 is operated to suck indoor air to be air-conditioned from the air suction port 431 in the indoor unit 402, the air is heat-exchanged in the indoor heat exchanger 421 and then the cooled air is supplied to the air filtering unit 404 (Fig. 9). The air supplied to the air filtering unit 404 is blown out from the air blow-out port 432 into the room to be air-conditioned.

On the other hand, when the heating operation is carried out, the controller 408 switches the four-way valve 413 to the heating side so that the refrigerant flows into the refrigerant circuit 500a, the outdoor heat exchanger 414 serves as an evaporator and the indoor heat exchanger 421 serves as a condenser as indicated by a solid-line arrow of Fig. 6. Then, the air blowing fan 422 is operated to suck indoor air of the room to be air-conditioned from the air suction port 431 in the indoor unit 402, the air is heat-exchanged in the indoor heat exchanger 421, and the heated air is supplied to the air filtering unit 404 (Fig. 9). The air supplied to the air filtering unit 404 is blown out from the air blow-out port 432 into the room to be air-conditioned.

As described above, at the same time when the air conditioning operation is carried out, the electrical component board 440 opens the water control valve 446 to supply tap water or the like to the electrolytic unit 405. Then, tap water or the like which is naturally or artificially added with a predetermined amount of ion species is electrolyzed in the electrolytic unit to generate electrolytic water containing a predetermined active oxygen species such as hypochlorous acid or the like, and the generated electrolytic water is supplied to the air filtering unit 404. The electrolytic water supplied to the air filtering unit 404 infiltrates into the gas-liquid contact member 441. At this time, the air supplied to the gas-liquid contact member 441 is brought into contact with the electrolytic water containing the active oxygen species in the air filtering unit 404, thereby filtering the air, that is, inactivating, sterilizing, decomposing or the like the virus, etc. contained in the air. The electrolytic water infiltrating into the gas-liquid contact member 441 is dropped to the water receiving tray 443 and stocked there.

In the second embodiment, even when the water control valve 446 is closed to stop supply of tap water or the like to the electrolytic unit 405, the electrical component board 440 can supply the electrolytic water stocked in the water receiving tray 443 to the electrolytic unit 405 by driving the circulating pump 449. Accordingly, even when the electrolytic unit 405 is stopped, electrolytic water is supplied to the air filtering unit 404. At this time, when the float switch 454 detects the low water-level state, the electrical component board 440 opens the water control valve 446 and supplies tap water or the like to the electrolytic unit 405.

The electrolytic water stocked in the water receiving tray 443 flows from the lower end of the drain pipe 444 to the drain pan 424 of the indoor heat exchanger 421 in connection with the opening of the flow rate control valve 445 under the control of the electrical component board 440. Here, the electrolytic water stocked in the water receiving tray 443 can be made to flow to the drain pan 424 intermittently (for example, every hour or the like) by intermittently opening/closing the valve 45 or to flow to the drain pan 424 at all times by opening the valve at all times. The electrolytic water dropping to the drain pan 424 is discharged to the outside by the drain pump 427.

According to the second embodiment, the flow rate (amount)control valve 445 is provided to the lower portion of the water receiving tray 443, and the water receiving tray 443 is connected to the drain pipe 444 provided to the upper side of the train pan 424 through the flow rate (amount) control valve 445. Accordingly, when the flow rate control valve 445 is opened, the electrolytic water stocked in the water receiving tray 443 flows through the drain pipe 444 and drops to the drain pan 424. Therefore, electrolytic water can be supplied to the drain pan 424 while a fixed amount of electrolytic water is stocked in the water receiving tray 443, thereby preventing breeding of various bacteria, fungus, etc. in the drain pan 424.

Furthermore, according to the second embodiment, there are provided the water receiving tray 443 for stocking electrolytic water dropped from the gas-liquid contact member (humidifying element) 441 and the circulating pump 449 for circulating the electrolytic water stocked in the water receiving tray 443 through the electrolytic unit 405 to the air filtering unit 404. Therefore, the electrolytic water generated in the electrolytic unit 405 can be saved, and tap water or the like supplied to the electrolytic unit 405 can be saved.

At this time, under the control of the electrical component board 440, the electrolytic water stocked in the water receiving tray 443 is intermittently dropped to the drain pan 424, whereby the electrolytic water to be circulated in the air filtering unit 404 can be saved with prevent ing breeding of various bacterial, etc. in the drain pan 424. At this time, the water receiving tray 443 is equipped with the float switch 454 When the float switch detects the low water level, the electrical component board 440 opens the water control valve 446 to supply tap water or the like to the electrolytic unit 405, so that electrolytic water required to be circulated to the air filtering unit 404 can be prevented from running short, and the water amount of the electrolytic water can be kept.

Furthermore, according to the second embodiment, the filtering unit 403 is equipped with the electrical component board 440, and it can be easily added to the indoor unit 402. In the second embodiment, three air filtering units 403 at maximum can be secured to the indoor unit 402.

### (Third Embodiment)

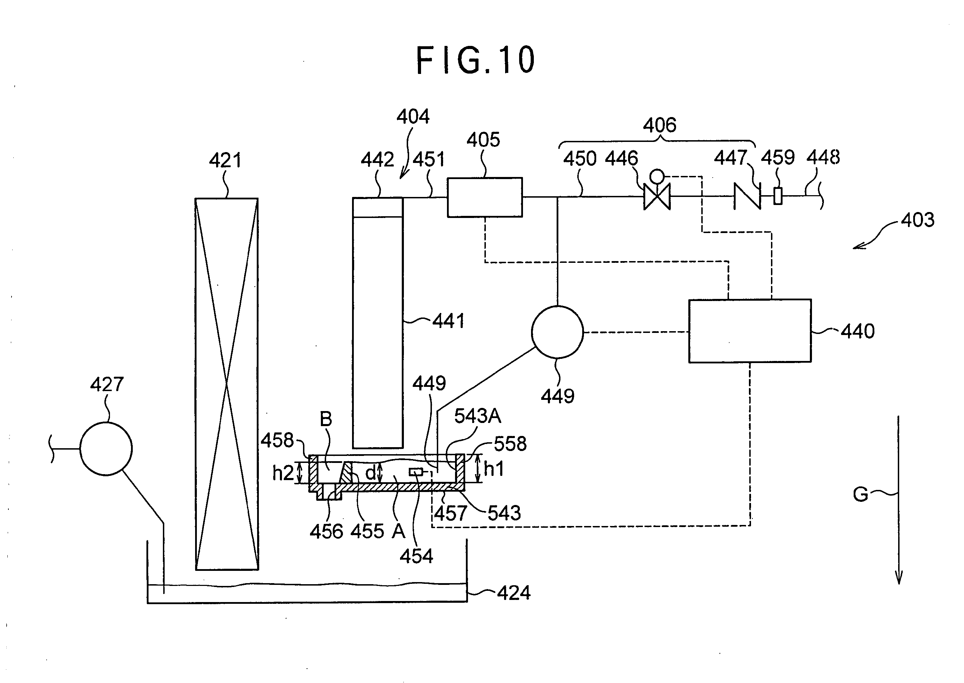

Fig. 10 shows a systematic diagram showing the flow of electrolytic water passed through the air filtering unit 404 of the third embodiment. An air conditioner 500 according to the third embodiment has the same construction as the second embodiment except for the construction associated with the water receiving tray 443, the flow rate control valve 445 and the drain pipe 444.

In the third embodiment, a water receiving tray 543 is provided below the gas-liquid contact element (humidifying element) 441.

The water receiving tray 543 includes a flat plate 457 disposed in a substantially horizontal position, and a wall portion 458 extending upwardly from the outer periphery of the flat plate 457 so as to form a tray-shaped (or dish-shaped) receiving portion 543A. A dam portion 455 having a low height h2 than the height h1 of the wall portion 458 is formed between confronting portions of the wall portion 458 so that the receiving portion 543A is divided to a water stock portion A and a drain portion B through the dam portion 455 as shown in Fig. 10.

The water stock portion A is provided beneath the gas-liquid contact member 441 so that electrolytic water dropped from the gas-liquidcontactmember 441 is stocked in the water stockportion A. Furthermore, a suction nozzle 449A of the circulating pump 449 is disposed in the water stock portion A. Accordingly, by driving the circulating pump 449, the electrolytic water stocked in the water stock portion A of the water receiving tray 543 is supplied to the electrolytic unit 405.

A drain port 456 is formed in the flat plate 457 at the drain portion B. The drain port 456 is designed so that the electrolytic water in the drain portion B is dropped to the drain pan 424 for the indoor heat exchanger 421. Accordingly, the electrolytic water flowing to the drain portion B is dropped through the drain port 456 to the drain pan 424.

Next, the operation of the air conditioner 500 of the third embodiment will be described.

The air conditioner 500 of the third embodiment carries out the same air-conditioning operation as the second embodiment, and the electrical component board 440 opens the water control valve 446 and supplies tap water or the like to the electrolytic unit 405 at the same time when the air-conditioning operation is carried out. Tap water or the like added with a predetermined amount of ion species is electrolyzed in the electrolytic unit 405 to generate electrolytic water containing a predetermined active oxygen species such as hypochlorous acid or the like, and the generated electrolytic water is supplied to the air filtering unit 404. Accordingly, the electrolytic water supplied to the air filtering unit 404 infiltrates into the gas-liquid contact member 441. At this time, in the air filtering unit 404, air supplied to the gas-liquid contact member 441 is brought into contact with the electrolytic water containing the active oxygen species and subjected to filtering. The electrolytic water infiltrating in the gas-liquid contact member 441 is dropped to the water stock portion A of the water receiving tray 543 and stocked there.

In the third embodiment, even when the water control valve 446 is closed and the supply of tap water or the like to the electrolytic unit 405 is stopped, the electrical component board 440 can also supply the electrolytic water stocked in the water stock portion A of the water receiving tray 543 to the electrolytic unit 405 by driving the circulating pump 449. Accordingly, even when the electrolytic unit 405 is stopped, electrolytic water is supplied to the air filtering unit 404. At this time, when the float switch 454 detects the low water level, the electrical component board 440 opens the water control valve 446 to supply tap water or the like to the electrolytic unit 405.

Furthermore, according to the third embodiment, the water receiving tray 543 is provided with the dam portion 455 which is lower than the wall portion 458. Therefore, when the water level d of the electrolytic water stocked in the water receiving tray 543 reaches the height h2 of the dam portion 455, the electrolytic water flows over the upper portion of the dam portion 455 to the drain portion B every time electrolytic water is newly dropped from the gas-liquid contact member 441 to the water stock portion A. That is, the reservoir capacity of the water stock portion A is determined by the height h2 of the dam portion 455, and electrolytic water whose amount corresponds to the excess amount of electrolytic water from the reservoir capacity flows to the drain portion B. Accordingly, the electrolytic water flowing to the drain portion B flows through the drain port 456 and drops to the drain pan 424 below the indoor heat exchanger 421. The electrolytic water dropped to the drain pan 424 is discharged to the outside together with drain water stocked in the drain pan 424 by the drain pump 426.

In the third embodiment, the water receiving tray 543 is divided into the water stock portion A and the drain portion B through the dam portion 455, and when the amount of electrolytic water stocked in the water stock portion A exceeds the reservoir capacity of the water stock portion A, electrolytic water dropped from the gas-liquid contact member 441 flows over the dam portion 455 to the drain portion B, and then flows to the drain pan 424. Accordingly, the electrical component board 440 opens the water control valve 446 to supply electrolytic water at all times so that the electrolytic water in the water stock portion A exceeds the reservoir capacity, whereby electrolytic water can be supplied to the drain pan 424. Or, the electrical component board 440 intermittently opens the water control valve 446 to intermittently supply electrolytic watersothatthe electrolytic water in the water stock portion A exceeds the reservoir capacity, whereby the electrolytic water can be supplied to the drain pan 424 while a fixed amount of electrolytic water is stocked in the water stock portion A. Therefore, various bacteria, virus, fungus, etc. can be prevented from breeding in the drain pan 424 without using any magnetic valve or the like in the water receiving tray 543.

Furthermore, the third embodiment is equipped with the water receiving tray (electrolytic water tray) 543 for stocking electrolytic dropped from the gas-liquid contact member (humidifying element) 441 and also the circulating pump 449 for circulating the electrolytic water stocked in the water receiving tray 543 to the air filtering unit 404 through the electrolytic unit 405. Therefore, the electrolytic water generated in the electrolytic unit 405 can be saved, and tap water or the like supplied to the electrolytic unit 405 can be also saved. In this case, the water receiving tray 543 is equipped with the float switch 454, and when the float switch 454 detects the low water level, the electrical component board 440 opens the water control valve 446 and supplies tap water or the like to the electrolytic unit 405. Therefore, the electrolytic water required to be circulated to the air filtering unit 404 does not run short, and the amount of electrolytic water can be maintained.

Furthermore, in the third embodiment, the filtering unit 403 is equipped with the electrical component board 440, and thus it can be easily added to the indoor unit 402. Accordingly, three filtering units 403 at maximum can be secured to the indoor unit 402 as in the case of the second embodiment.

In the above third embodiment, hypochlorous acid is generated as the active oxygen species. However, in place of hypochlorous acid, ozone (O₃) or hydrogen peroxide (H₂O₂) may be generated as the active oxygen species. In this case, if platinum tantalum electrodes are used as the electrodes in the electrolytic unit 405, the active oxygen species can be highly efficiently and stably generated from even ion-species rare water by electrolysis.
At this time, at the anode, the following reaction occurs:

2H₂O → 4H⁺ + O₂ + 4e⁻

At the same time, the following reactions occur, and ozone (O₃) is generated:

3H₂O → O₃ + 6H⁺ + 6e⁻

2H₂O → O₃ + 4H⁺ + 4e⁻

At the cathode, O₂⁻ generated through the electrode reaction and H⁺ in the solution react with each other, and hydrogen peroxide (H₂O₂) is generated according to the following reactions:

4H⁺ + 4e⁻ + (4OH) → 2H₂ + (4OH)

+ e⁻ + 2H⁺ → H₂O₂

In this construction, by supplying current to the electrodes in the electrolytic unit 405, ozone (O₃) and hydrogen peroxide (H₂O₂) having strong sterilizing power occur, and electrolytic water containing ozone (O₃) and hydrogen peroxide (H₂O₂) is generated. The concentration of ozone or hydrogen peroxide in the electrolytic water is adjusted to such a concentration that target virus, etc. can be inactivated, and air is passed through the gas-liquid contact member 441 supplied with the electrolytic water of this concentration, whereby the target virus, etc. floating in the air can be inactivated. Furthermore, when gaseous material such as odor or the like passes through the gas-liquid contact member 441, it is dissolved in the electrolyticwater or it reacts with ozone or hydrogen peroxide in the electrolytic water, so that these materials are removed from the air and thus deodorized.

Furthermore, in the third embodiment, the water stock portion A and the drain portion B are surrounded by the wall portion 458, and the electrolytic water flowing from the water stock portion A over the dam portion 455 to the drain portion B is passed through the drain port 456 formed in the drain portion B, and dropped to the drain pan 424. However, the present invention is not limited to this embodiment. For example, the electrolytic water flowing from the water stock portion A over the dam portion 455 may be directly dropped to the drain pan 424 with providing neither the drain portion B nor the drain port 56.

Furthermore, the third embodiment relates to the four-way blow-out type in-ceiling embedded air conditioner. However, the air conditioner is not limited to this type, but it may be applied to one-way or two-way air conditioner. Furthermore, it is not limited to the in-ceiling embedded type, and for example, it may be a ceiling-suspended type, a wall-suspended type, an on-floor mount type or the like.

In the third embodiment, the electrolytic water is passed from the lower portion of the water receiving tray 443 through the drain pipe 444 and dropped to the drain pan 424, or the electrolytic water flowing over the upper portion of the dam portion 455 provided to the water receiving tray 543 is dropped to the drain pan 424. However, the present invention is not limited to this embodiment, and any construction may be adopted insofar as the electrolytic water supplied to the gas-liquid contact member 441 is supplied to the drain pan 424. For example, the electrolytic water stocked in the water receiving tray may be forcedly supplied to the drain pan 424 by a pump or the like.

### (Fourth Embodiment)

A fourth embodiment of the present invention will be described with reference to the accompanying drawings.

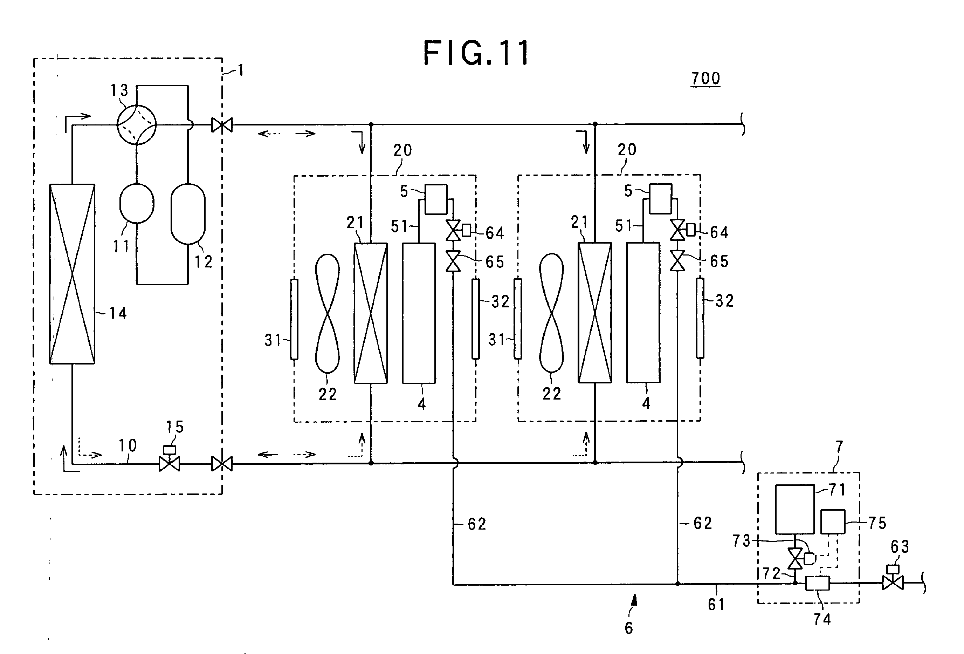

Fig. 11 shows the construction of an air conditioning system 700 according to a fourth embodiment of the present invention. The air conditioning system 700 of the fourth embodiment has a so-called multiple type air conditioner 700a including one outdoor unit 601 and plural indoor units 602 connected to the outdoor unit 601. The number of the indoor units 602 is not limited to a specific value, and only one indoor unit 602 may be provided.

The refrigerant circuit of the air conditioner 700a of this embodiment is basically the same as that of the first embodiment shown in Fig. 1 except for provision of a controller 75 for adjusting the opening degree or the flow rate (amount) of the flow rate (amount) adjusting valve 73 on the basis of the electrical conductivity of tap water or the like detected by the electrical conductivity meter 74 to control the amount of ion species to be added to the tap water or the like. Therefore, the duplicative description thereof is omitted from the following description, and the same elements or corresponding elements are represented by the same reference numerals.

The ion species to be added to tap water or the like is the same as the first embodiment. In the first embodiment, the concentration of the ion species such as chloride ions or the like is estimated on the basis of the electrical conductivity of tap water or the like supplied from the water supply source, and a predetermined ion species is added in accordance with the estimated concentration concerned. In the fourth embodiment, a predetermined ion species may be added in accordance with the concentration of the ion species or the electrical conductivity of tap water or the like which is set (stored, registered) in the controller 675 or the like. At this time, when the concentration of the ion species such as tap water or the like supplied from the water supply source is less than a predetermined concentration (for example, 30ppm or less in chloride ions or the like), the controller 75 may control the valve opening degree of the flow rate adjusting valve 73 so that a predetermined amount of ion species is added, or may adjust the valve opening degree of the flow rate adjusting valve 73 to the valve opening degree corresponding to variation of the electrical conductivity so that the concentration of the ion species in tap water or the like supplied to the electrolytic unit 5 is equal to a predetermined value.

Furthermore, the construction of the indoor unit of this fourth embodiment is basically the same as that of the first or second embodiment shown in Figs. 2, 3, 7 and 8, and the constructions of the air filtering unit and the electrolytic unit of the fourth embodiment are also basically the same as those of the first or second embodiment shown in Figs. 4A, 4B and 8. Accordingly, the detailed descriptions of these constructions are omitted from the following description, and only the different points will be described hereunder.

Next, the operation of the air conditioner according to the fourth embodiment will be described with reference to Fig. 11.

When an operation start instruction is input from an indoor remote controller (not shown) or the like by a user, the controller 75 switches the four-way valve 13 of the outdoor unit 1 to a cooling side or heating side in accordance with the operation mode (cooling operation mode/heating operation mode) indicated by the operation start instruction to carry out a predetermined air-conditioning operation such as a cooling operation, a heating operation or the like.

When the cooling operation is carried out, the controller 75 switches the four-way valve 13 to the cooling side so that refrigerant flows into the refrigerant circuit, the outdoor heat exchanger 14 served as a condenser and the indoor heat exchanger 21 serves as an evaporator as indicated by a broken-line arrow of Fig. 11. Then, the air blowing fan 22 is operated to suck indoor air to be air-conditioned from the air suction port 31 in the indoor unit 2, the air is heat-exchanged in the indoor heat exchanger 21 and then the cooled air is supplied to the air filtering unit 4 (Fig. 11). The air supplied to the air filtering unit 4 is blown out from the air blow-out port 32 into the room to be air-conditioned.

On the other hand, when the heating operation is carried out, the controller 75 switches the four-way valve 13 to the heating side so that the refrigerant flows into the refrigerant circuit, the outdoor heat exchanger 14 serves as an evaporator and the indoor heat exchanger 21 serves as a condenser as indicated by a solid-line arrow of Fig. 11. Then, the air blowing fan 22 is operated to suck indoor air of the room to be air-conditioned from the air suction port 31 in the indoor unit 2, the air is heat-exchanged in the indoor heat exchanger 21, and the heated air is supplied to the air filtering unit 4 (Fig. 11) . The air supplied to the air filtering unit 4 is blown out from the air blow-out port 32 into the room to be air-conditioned.

At the same time when the air conditioning operation is carried out, the controller 75 opens the opening/closing valves 63 and 64 to supply tap water or the like to the electrolytic unit 5. At this time, the electrical conductivity of the tap water or the like supplied from the water supply source is detected by the electrical conductivity meter 74. When the concentration of the ion species estimated on the basis of the detected electrical conductivity is lower than a predetermined value, the opening degree of the flow rate (amount) adjusting valve 73 is adjusted, and a predetermined amount of (material containing) the ion species is dropped to the tap water or the like. Then, the tap water or the like which is added with the predetermined amount of the ion species is electrolyzed in the electrolytic unit 5 to generate electrolytic water containing a predetermined active oxygen species such as hypochlorous acid or the like. The generated electrolytic water is supplied to the air filtering unit 4. In the air filtering unit 4, the air supplied to the gas-liquid contact member 41 is brought into contact with the electrolytic water containing the active oxygen species and filtered (inactivated, sterilized, etc.).

According to the fourth embodiment described above, air introduced into the indoor unit 2 by the air blowing fan 22 is heat-exchanged by the indoor heat exchanger 21 and then blown out to the room. Furthermore, air introduced into the indoor unit 2 by the air blowing fan 22 is also subjected to air filtering in the air filtering unit 4 by bringing the air into contact with electrolytic water containing active oxygen species. The air filtering unit 4 is supplied with electrolytic water containing active oxygen species generated in the electrolytic unit 5. The electrolytic unit 5 is connected to a water pipe, a water supply source such as a water supply tank or the like through a water distributing pipe 6, so that the electrolytic unit 5 is supplied with water such as tap water, well water or the like from the water supply source. The water stock tank (ion-species supply unit) 71 for adding ion species to water supplied from the water supply source is provided to the water distributing pipe 6. Therefore, water containing ion species is supplied to the electrolytic unit 5 irrespective of the concentration of the ion species of the water supplied from the water supply source, so that the electrolytic water can be efficiently generated and the air can be efficiently filtered by the generated electrolytic water.

For example, when influenza virus invades into indoor air, the active oxygen species functions to break down and vanish (remove) the surface protein (spike) of the virus concerned which is indispensable for infection. When the surface protein of influenza virus is broken down, the influenza virus is not joined to a receptor which is necessary for infection of the virus concerned, so that infection can be prevented. Therefore, influenza virus floating in the air is brought into contact with the electrolytic water containing the active oxygen species in the gas-liquid contact member 41, so that the influenza virus loses so-called infection power, and thus the infection can be prevented. As a result of a verification test which was made in cooperation with Sanitary Environment Research, it has been found that when air in which influenza virus invades is passed through the gas-liquid contact member 31 of this embodiment, 99% or more of the virus concerned can be removed.

In the fourth embodiment, the water distributing pipe 6 is constructed by the water supply source connecting pipe 61 ad the water distributing pipe 62 that is branched from the water supply source connecting pipe 61 and distributes water to each unit, and the ion species adding unit 7 is constructed to add ion species in a lump in the water supply source connecting pipe 61. Therefore, ion species of a predetermined concentration or more is added to tap water or the like distributed to the electrolytic units 5 of the respective indoor units 2 through the respective water distributing pipe 62, and the electrolytic water can be efficiently generated in the respective electrolytic units 5. Furthermore, when plural indoor units 20 are provided in connection with one indoor unit 1 in the air conditioning system 700, the ion species adding unit 7 is provided to the water supply source connecting pipe 61, and thus it is unnecessary to provided the ion species adding unit 7 every indoor unit 2. Therefore, the construction of the air conditioning system 700 can be simplified. Furthermore, it is unnecessary to supplement material containing a predetermined ion species or the like every indoor unit 2.

According to the air conditioning system 700 of the fourth embodiment, the air flowing passage is formed by the air blowing fan 22 so as to extend from the air suction port 31 to the air blow-out port 32, and the air filtering unit 4 is disposed at the downstream side of the indoor heat exchanger 21 on the air flowing passage, whereby the following effect can be achieved. That is, under the cooling operation of the air conditioning system 700 of the fourth embodiment, air is cooled by the indoor heat exchanger 21 and air having relatively high humidity is supplied to the air filtering unit 4. Under the heating operation of the air conditioning system 700, air is heated by the indoor heat exchanger 21, and air having relatively low humidity is supplied to the air filtering unit 4.

Accordingly, even when the supplied air is brought into contact with electrolytic water in the air filtering unit 4, the relative humidity of the air filtered in the air filtering unit 4 can be prevented from increasing under cooling operation because the air supplied to the air filtering unit 4 has already had relatively high humidity under cooling operation. On the other hand, under heating operation, the air having relatively low humidity is supplied to the air filtering unit 4, and thus the relative humidity of the air filtered in the air filtering unit 4 can be increased by bringing the air into contact with the electrolytic water. Accordingly, by merely filtering/cleaning air in a wet mode and also switching the four-way valve 13 to switch the cooling operation and the heating operation to each other, the humidification amount of air under air conditioning operation can be automatically controlled, and the indoor air environment can be kept comfortable.

Furthermore, under cooling operation, heat-exchanged air can be filtered in the air filtering unit 4 and clean air can be supplied to the room. In addition, the relative humidity of the air supplied to the air filtering unit is high, and thus the consumption of the electrolytic water can be suppressed.

According to the fourth embodiment, the electrolytic water containing active oxygen species such as hypochlorous acid or the like is discharged from the water receiving tray 43 disposed below the air filtering unit 4 through the drain pipe 44 to the drain pan 24 (see Fig. 4A) . Therefore, the electrolytic water is contaminated into the drain water stocked in the drain pan 24, so that occurrence of various bacteria, fungus, etc. in the drain water can be prevented, and thus occurrence of slime on the drain pan 25 can be prevented. Therefore, the frequency of the cleaning and maintenance of the drain pan 24 can be reduced, and the labor imposed on the cleaning and maintenance works can be reduced.

According to the fourth embodiment, the air filtering unit 4 is provided at the air blow-out port 32 side of the indoor unit 2, and thus water contained in air blown out from the air filtering unit 4 is prevented from being directly introduced into the indoor heat exchanger 21. Therefore, corrosion of the indoor heat exchanger 21 can be prevented from being promoted by water.

Various modifications may be properly made to the fourth embodiment without departing from the subject matter of the present invention. In the fourth embodiment, the valve opening degree of the flow rate adjusting valve 73 is adjusted on the basis of the electrical conductivity of tap water or the like to control the amount of ion species to be added to the tap water or the like. However, when variation of the concentration of predetermined ion species such as chloride ions or the like in tap water or the like (variation of the electrical conductivity of tap water or the like) is little, the concentration of the ion species may be measured in advance when the air conditioner is set up, and the valve opening degree of the flow rate adjusting valve 73 which corresponds to the measured concentration of the ion species is set in advance. Furthermore, in the above construction, detection of the electrical conductivity of tap water or the like may be carried out at the time when the electrolysis of tap water or the like is started. However, since the electrical conductivity of tap water or the like is not greatly varied over a day, the electrical conductivity may be detected not every time the electrolysis is carried out, but once after the electrolysis is carried out several times.

Furthermore, the fourth embodiment relates to a so-called in-ceiling embedded type four-way blow-out type indoor unit 2. However, the indoor unit of the present invention is not limited to this type, and it may be a ceiling-suspended type, a wall-suspended type or an on-floor mount type. Still furthermore, in the fourth embodiment, the air filtering unit 4 is dispose between the thermal insulating member 23 and one side of the substantially rectangularly bent indoor heat exchanger 21 disposed in the housing 20 of the indoor unit 2. However, it is needless to say that the air filtering unit 4 may be disposed along one or more sides of the heat exchanger 21. Furthermore, the gas-liquid contact member 41 of the air filtering unit 4 may be disposed substantially in parallel to or obliquely to the indoor heat exchanger 21.

Furthermore, in the above embodiment, the stock tank 71 is disposed at a low place in a room by mounting the stock tank 71 on the floor or the like, and connected through the distributing pipe 72 to the water supply source connecting pipe 61 disposed under floor or the like, however, the present invention is not limited to this embodiment. For example, the stock tank 71 may be disposed under the roof or the like in the in-ceiling embedded type air conditioner 700a. In this case, it is preferable that the stock amount of ion species in the stock tank 71 is set to such an amount that the air conditioner 700a can be continuously operated for one year or half a year.

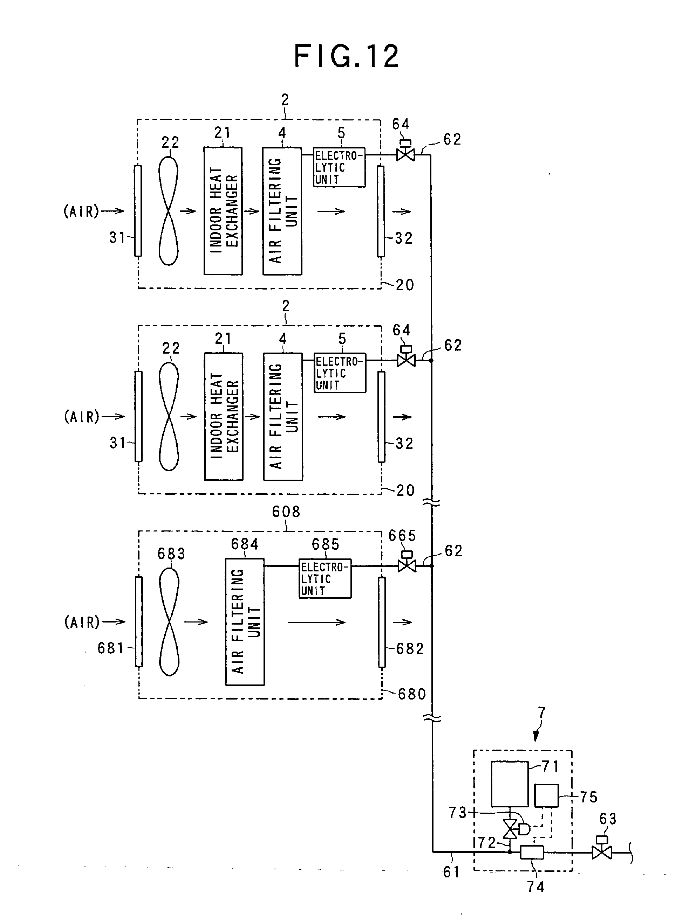

As described above, the fourth embodiment relates to the air conditioning system 700 including plural indoor units 2 (for example, two) for one outdoor unit 1. However, one or plural air filtering apparatuses 608 may be added to the above air conditioning system 700 of the fourth embodiment as shown in Fig. 12.

The air filtering apparatus shown in Fig. 12 is equipped with a housing 680 having an air suction port 681 and an air blow-out port 682, an air blowing fan 683 for blowing air sucked from the air suction port 681 to the air blow-out port 682, an air filtering unit 684 which is disposed on an air flowing passage formed in the housing 680 by the air blowing fan 683 and brings air supplied through the air flowing passage into contact with electrolytic water containing an active oxygen species to filter the air, and an electrolytic unit 685 (electrolytic water supply unit) for generating the electrolytic water containing the active oxygen species by electrolyzing water containing a predetermined ion species and supplying the generated electrolytic water to the air filtering unit 684. In Fig. 12, the same constituent elements as the fourth embodiment are represented by the same reference numerals, and the description thereof is omitted.

As shown in Fig. 12, the water distributing pipe 62 branched from the water supply source connecting pipe (water common pipe) 61 is also connected to the electrolytic unit 685 of the air filtering apparatus 608. When the concentration of ion species of tap water or the like supplied through the water distributing pipe 62 is low, a predetermined ion species is added to the tap water or the like by the ion species adding unit 7. An opening/closing valve 665 is provided to the water distributing pipe 62 in the air filtering apparatus 608.

In the construction shown in Fig. 12, when tap water or the like is supplied to each of the electrolytic units 5 and 685 of the plural indoor units 2 and the air filtering apparatus 608, the ion species can be collectively added in the water supply source connecting pipe 61, and thus the electrolytic water can be efficiently generated in each of the electrolytic units 5 and 685. Furthermore, even when the plural indoor units 2 and the air filtering apparatus 608 are provided, the ion species adding unit 7 is provided to the water supply source connecting pipe 61, and thus it is unnecessary to individually provide the ion species adding unit 7 to each of the indoor units 2 and the air filtering apparatus 608, and thus the construction of the air conditioning system of this embodiment can be simplified. Furthermore, it is unnecessary to individually supplement (material containing) a predetermined ion species to each of the indoor units 2 and the air filtering apparatus 608, and thus the maintenance can be easily performed.

If the plural indoor units 2 are replaced by plural air filtering apparatuses 608 in the above air conditioning system 700, the air conditioning system is changed to an air filtering system. In this case, the air filtering unit 684 and the electrolytic unit 5 provided to each air filtering apparatus 8 maybe designed to have the same construction as the air filtering unit 4 and the electrolytic unit 5 provided to the indoor unit 2.

Furthermore, in the above fourth embodiment, hypochlorous acid is generated and used as the active oxygen species. However, ozone (O₃) or hydrogen peroxide (H₂O₂) may be generated and used as the active oxygen species. In this case, if platinum tantalum electrodes are used as the electrodes in the electrolytic unit 5, the active oxygen species can be highly efficiently and stably generated from even ion-species rare water by electrolysis. Therefore, the amount of ion species to be added can be reduced in the ion species adding unit 7.

At this time, at the anode, the following reaction occurs:

2H₂O → 4H⁺ + O₂ + 4e⁻

At the same time, the following reactions occur, and ozone (O₃) is generated:

3H₂O → O₃ + 6H⁺ + 6e⁻

2H₂O → O₃ + 4H⁺ + 4e⁻

At the cathode, O₂⁻ generated through the electrode reaction and H⁺ in the solution react with each other, and hydrogen peroxide (H₂O₂) is generated according to the following reactions:

4H⁺ + 4e⁻ + (4OH) → 2H₂ + (4OH)

O₂⁻ + e⁻ + 2H⁺ → H₂O₂

In this construction, by supplying current to the electrodes in the electrolytic unit 5, ozone (O₃) and hydrogen peroxide (H₂O₂) having strong sterilizing power occur, and electrolytic water containing ozone (O₃) and hydrogen peroxide (H₂O₂) is generated. The concentration of ozone or hydrogen peroxide in the electrolytic water is adjusted to such a concentration that target virus, etc. can be inactivated, and air is passed through the gas-liquid contact member supplied with the electrolytic water of this concentration, whereby the target virus, etc. floating in the air can be inactivated. Furthermore, when gaseous material such as odor or the like passes through the gas-liquid contact member, it is dissolved in the electrolytic water or it reacts with ozone or hydrogen peroxide in the electrolytic water, so that these materials are removed from the air and thus deodorized.

Furthermore, when scales are deposited on the electrode (cathode) in the electrolytic unit 5 due to electrolysis of tap water or the like, the electrical conductivity is lowered or water flow to the electrolysis plane is disturbed or it is difficult to continuously conduct electrolysis. In this case, it is effective to invert the polarities of the electrodes (the plus and minus polarities of the electrodes are switched to each other). By conducting electrolysis while the cathode is set to the anode, scales deposited on the cathode can be removed. Under this polarity inverting control, the polarities of the electrodes may be inverted periodically by using a timer or the like, or irregularly inverted. Furthermore, increase of electrolysis resistance (reduction in electrolysis current or increase of electrolysis voltage) may be detected, and the polarities of the electrodes may be inverted on the basis of the detection result.

### (Fifth Embodiment)

A fifth embodiment according to the present invention will be described with reference to the accompanying drawings.

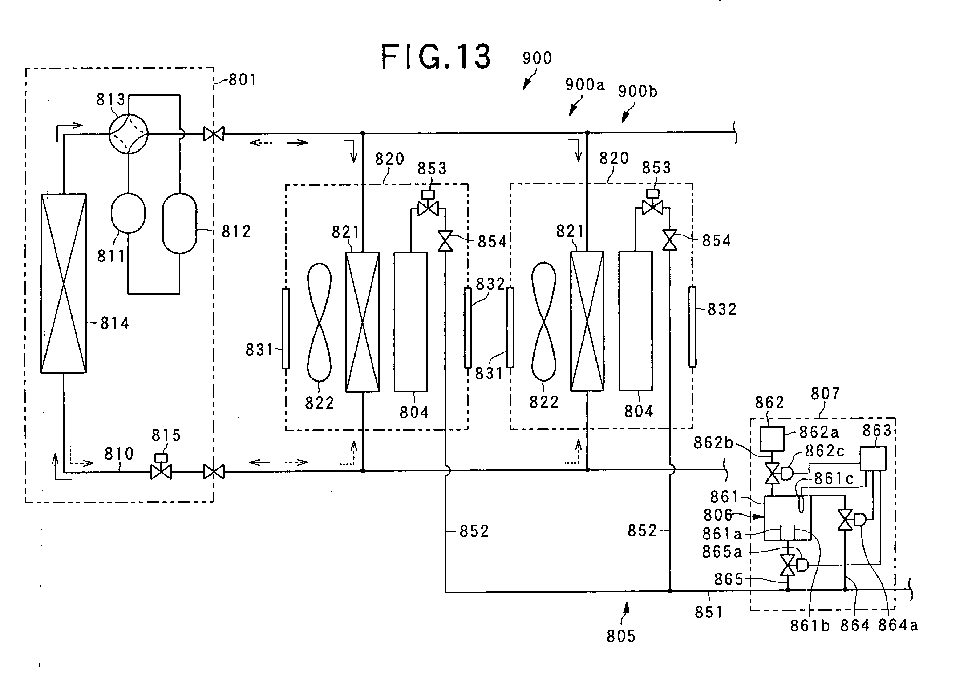

Fig. 13 shows the construction of an air conditioning system 900 according to the fifth embodiment of the present invention. The air conditioning system 900 of the fifth embodiment has a so-called multiple type air conditioner 900a in which plural indoor units 802 are connected to one outdoor unit 801.

The outdoor unit 801 is set up outdoors. In this outdoor unit 801, as shown in Fig. 13, a compressor 811 is disposed in a refrigerant pipe 810, an accumulator 812 is connected to the suction side of the compressor 811, and a four-way valve 813, an outdoor heat exchanger 814 and an electrically-driven expansion valve 815 are successively connected to the discharge side of the compressor 811 in this order. By switching the four-way valve 813, refrigerant flows in a direction indicated by a broken-line arrow in Fig. 13 under cooling operation, and also refrigerant flows in a direction indicated by a solid-line arrow in Fig. 13 under heating operation, whereby the cooling operation and the heating operation are switched to each other.

Each indoor unit 802 is set up indoors, and it is connected to the outdoor unit 801 through a refrigerant pipe 810. As shown in Fig. 13, an indoor heat exchanger 821, an air blowing fan 822 for introducing air through an air suction port 831 into the indoor unit 802 and blowing air heat-exchanged in the indoor heat exchanger 821 through an air blow-out port 832 to a room, and an air filtering unit 804 for bringing air introduced into the indoor unit 802 by the air blowing fan 822 into contact with electrolytic water containing active oxygen species to filter the air.

The air filtering unit 804 of each indoor unit 802 is connected to an external water supply source (not shown) through a water distributing pipe 805. An electrolytic unit 806 is provided in the water distributing passage of the water distributing pipe 805. The electronic unit 806 generates high-concentration electrolytic water containing active oxygen species of high concentration, and adjusts the mixing ratio of the high-concentration electrolytic water and water supplied from the external water supply source by adding the generated electrolytic water to the water, thereby preparing electrolytic water containing the active oxygen species of a desired concentration. The electrolytic water whose concentration is adjusted to a desired value in accordance with the operation mode is supplied through the water distributing pipe 805 to each air filtering unit 804.

The water distributing pipe 805 is equipped with a water supply source connecting pipe (common water pipe) 851 that is provided with the electrolytic unit 806 and connected to the external water supply source, and a water distributing pipe 852 that is branched from the water supply source connecting pipe 851 to each indoor unit 802 to distribute electrolytic water to each air filtering unit 804. Furthermore, an opening/closing valve 853 is provided to each water distributing pipe 852, and electrolytic water is supplied to each air filtering unit 804 by opening/closing the corresponding opening/closing valve 853. Furthermore, a check valve 854 is provided at the upstream side of the opening/closing valve 853 to prevent backflow of electrolytic water.

Here, water supplied from the external water supply source is not limited to specific one. For example, a water pipe may be connected to the water distributing pipe to supply city water (tap water) as the water supply source. Or, a water supply tank or the like may be used as the water supply source so that water stocked in the water supply tank or the like is supplied. The water stocked in the water supply tank or the like may be water containing a predetermined ion species such as chloride ions or the like in advance such as tap water or the like, or water containing an ion species of low concentration such as well water or the like. Water supplied from the water supply source through the water distributing pipe 805 will be referred to as "tap water or the like".

In the fifth embodiment, the same active oxygen species as described with reference to the first to fourth embodiments are used. That is, the active oxygen species means oxygen molecules having higher oxidizing activity than normal oxygen and also related substance thereof, and contain not only so-called narrowly-defined active oxygen such as superoxide anion, singlet oxygen, hydroxyl radical and hydrogen peroxide, but also so-called broadly-defined active oxygen such as ozone, hypochlorous acid, hypohalous acid, etc.

Furthermore, a high-concentration electrolytic water is added to the water distributing pipe 805, and thus it is preferable that the water distributing pipe 805 is formed of a material that has resistance to electrolytic water containing active oxygen species such as hypochlorous acid, etc. and does not induce deterioration of the water distributing pipe 805 such as rust, etc. A plastic pipe or a composite pipe whose inside is formed of iron and coated with plastic material may be used for the water distributing pipe 805. For example, a chloride vinyl pipe, a polyethylene pipe, a polybutene pipe, a cross-linked polyethylene pipe or the like may be used as the plastic pipe. Furthermore, a resin lining steel pipe or the like may be used as the composite pipe.

As shown in Fig. 13, the electrolytic unit 806 includes an electrolytic bath 861 having at least a pair of electrodes 861a and 861b and a water level sensor 861c, an ion species adding unit 862, and a controller 863 for controlling various operations in the electrolytic unit 806 to efficiently generate high-concentration electrolytic water. The controller 863 can also serve as a controller for controlling the overall operation of the air conditioning system 900.

In the electrolytic bath 861, current is supplied to the electrodes 861a and 861b to electrolyze tap water or the like and generate high-concentration electrolytic water. The electrolytic bath 861 is connected to the water supply source connecting pipe (common pipe) 851 through a water supply pipe 864 and an electrolytic water drop pipe 865. The water supply pipe 864 is provided with an opening/closing valve 864a, and tap water or the like is introduced from the water supply source connecting pipe 851 into the electrolytic bath 861 by opening the opening/closing valve 864a. Furthermore, the electrolytic water drop pipe 865 is provided with a flow rate (amount) adjusting valve 865a, and the amount of the high-concentration electrolytic water to be dropped into the water distributing passage of the water supply source connecting pipe 851 is controlled by adjusting the valve opening degree of the flow rate adjusting valve 865a.

The electrodes 861a, 861b may be constructed by two electrode plates each of which comprises a base of Ti (titan) and a coated layer of Ir (iridium), Pt (platinum).

When current is supplied to tap water or the like by the electrodes 861a, 861b, the following reaction occurs at the cathode:

4H⁺ + 4e⁻ + (4OH⁻) → 2H₂ + (4OH⁻)

Furthermore, the following reaction occurs at the anode:

2H₂O → 4H⁺ + O₂ + 4e⁻

At the same time, chlorine ions or the like which are originally contained in tap water or the like or added by the electrolytic unit 806 reacts as follows:

2Cl⁻ → Cl₂ + 2e⁻

Furthermore, Cl₂ thus generated reacts with water as follows:

Cl₂ + H₂O → HClO + HCl

In this construction, by supplying current to the electrodes 861a, 861b, HClO (hypochlorous acid) having strong sterilizing power, etc. are generated. Therefore, air is bright into contact with electrolytic water in the air filtering unit 804 supplied with electrolytic water containing active oxygen species such as hypochlorous acid or the like to inactivate or sterilize virus, etc. floating in the air, thereby filtering the air. In addition, breeding of various bacteria, fungus, etc. in the air filtering unit 804 can be prevented. Furthermore, when gaseous materials such as odor components, etc. pass through the air filtering unit 804, the odor components are dissolved in the electrolytic water or also react with hypochlorous acid or the like in the filtering water, whereby the odor components can be removed from air and thus the air is deodorized.

The predetermined ion species is preferably halide ions (X⁻), and particularly preferably chloride ions (Cl⁻). When chloride ions are added to tap water or the like as the predetermined ion species, for example, natrium chloride (NaCl) or potassium chloride (KCl) may be used as the material containing chloride ions. When natrium chloride is used, commercially-sold salt or the like may be used, and this is preferable because it can be treated at low price and safely.

Electrolyte such as natrium chloride or the like is stocked in the stock tank 862a while it is adjusted in water solution like brine. By adding the predetermined ion species to tap water or the like under the water solution state, the predetermined ion species can be easily added to tap water or the like.

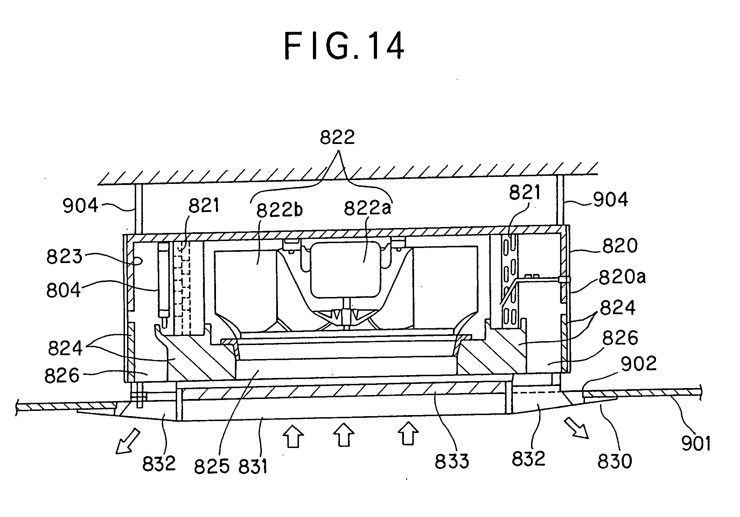

Next, the specific construction of the indoor unit 802 will be described with reference to Figs. 14.

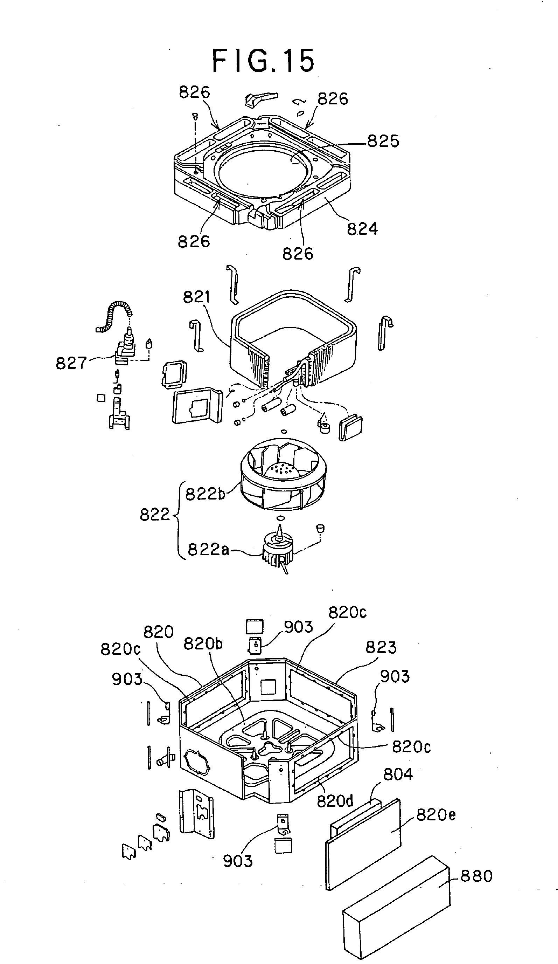

As shown in Figs. 14 and 15, the indoor unit 802 of the fifth embodiment is a so-called in-ceiling embedded type four-way blow-out type indoor unit 802, and the housing 820 is embedded in a ceiling hole 902 which is formed in a substantially rectangular form in the ceiling 901 of a building.

As shown in Fig. 15, the housing 820 is designed in a substantially rectangular box-shape which is opened at the lower surface thereof (the upper side in Fig. 15). Suspending tags 903 are provided at the four corners of the housing 820, and fixed to suspending bolts 904 suspended from the back side of the ceiling, whereby the housing 820 is suspended in the ceiling space.

A face panel 830 which is formed in a substantially rectangular (square) in plan view is provided to the lower surface of the housing 820, and the ceiling hole 902 is covered by the face panel 830. An air suction port 831 for sucking indoor air into the housing 820 is formed at the substantially center of the face panel 830 in plan view. A filter 833 is mounted inside the air suction port 831, that is, at the back side of the ceiling 901. Furthermore, air blow-out ports 832 are formed at the four sides of the face panel 830 so as to be elongated along the respective four sides, and air is blown out from the four air blow-out ports 832 in four directions in the room.

Next, the internal construction of the housing 820 will be described.

A thermal insulating member 823 formed of foamed polystyrene is provided to the inner surface of a side plate 820a of the housing 820. A motor 822a is fixed to the inside 820b of the top plate of the housing 820, and the shaft of the motor 822 is fixed to a vane wheel 822b. These elements constitute an air blowing fan 822. An indoor heat exchanger 821 which is bent in a substantially rectangular shape along the side plate 820a of the housing 820 is disposed inside the thermal insulating member 823 of foamed polystyrene so as to surround the air blowing fan 822 (Fig. 15) . Air sucked from the air suction port 831 by the air blowing fan 822 is supplied to the indoor heat exchanger 821, and air heat-exchanged in the indoor heat exchanger 821 is blown from each air blow-out port 832.

As shown in Fig. 14, a drain pan 824 formed of foamed polystyrene is disposed below the indoor heat exchanger 821. The drain pan 824 is disposed in the housing 820 while the outer peripheral surface thereof is substantially in contact with the inner surface of the housing 820. Furthermore, the drain pan 824 is provided with an air suction port 825 and air blow-out openings 826 at the positions corresponding to the air suction port 831 and the air blow-out ports 832 of the face panel 830. As shown in Fig. 15, the air suction opening 825 is formed in a substantially circular shape at the center of the substantially rectangular drain pan 824 in plan view. Furthermore, the air blow-out openings 826 are formed along the respective four sides of the drain pan 824.

A drain pump 827 is provided at the position corresponding to one corner of the indoor heat exchanger 821, and drain water stocked in the drain pan 824 is pumped up by a drain pump 827 (see Fig. 15), and discharged to the indoor unit 802.

Furthermore, an air filtering unit 804 is disposed at the outside of one side of the indoor heat exchanger 821 which is bent in a substantially rectangular shape. In the fifth embodiment, the air filtering unit 804 is disposed between the side corresponding to the endportion of the indoor heat exchanger 821 and the thermal insulating member 823. Accordingly, air filtered in the air filtering unit 804 is blown out from one air blow-out port 832 formed in the face panel 830. However, as shown in Fig. 15, knock-out portions 820c for securing the air filtering unit 804 are formed on the three side plates 820a out of the four side plates 820a forming the outer periphery of the housing 820. Any knock-out portion 820c is punched out in accordance with the setup position of the indoor unit 802 to form a hole 820d, and the air filtering unit 804 is accommodated in the housing 820 while the air filtering unit 804 is secured to a plate member 820e which covers the hole 820d. That is, the mount position of the air filtering unit 804 can be properly changed in accordance with the setup position of the indoor unit 802. Furthermore, plural air filtering units 804 may be accommodated in the indoor unit 802 by punching out plural knockout portions 820c to form plural holes 820d.

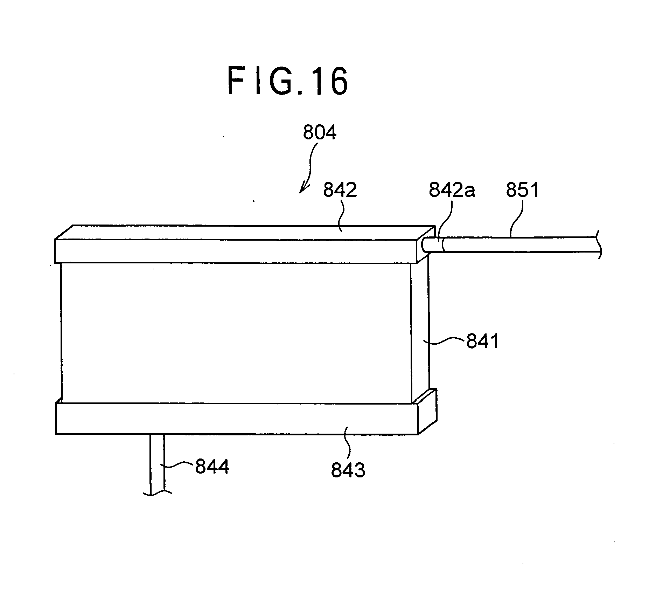

As shown in Fig. 16, the air filtering unit 804 is equipped with a gas-liquid contact member (humidifying element) 841 having high water retentivity, a water dispersing tray 842 disposed at the upper side of the gas-liquid contact member 841 and a water receiving tray (electrolytic water tray) 843 disposed at the lower side of the gas-liquid contact member 841.

The gas-liquid contact member 841 may be constructed by non-woven cloth formed of acrylic fiber, polyester fiber or the like. A raw material having little reactivity to electrolytic water is preferably used as the raw material of the gas-liquid contact member 841, and further other materials such as polyolefin-based resin (polyethylene resin, polypropylene resin or the like), vinyl chloride resin, fluorinated rein (PTFE, PFA, ETFE or the like), cellulose-based material, ceramics-based material, etc. may be used. Furthermore, the gas-liquid contact member 841 may be subjected to a hydrophilic treatment or the like to enhance the affinity of the gas-liquid contact member 841 to electrolytic water. Accordingly, the water retentivity (wettability) of the gas-liquid contact member 41 to electrolytic water is kept, and the contact between electrolytic water and introduced air can be kept for a long term.

The water dispersing tray 842 has a connection port 842a at the right side portion in the longitudinal direction, and an electrolytic water supply pipe (electrolytic water injecting tube) 851 is connected to the connection port 842a. Many holes (hot shown) for dropping electrolytic water supplied through the electrolytic water supply pipe 851 and dispersing the electrolytic water into the gas-liquid contact member 841 are formed in the bottom surface of the water dispersing tray 842.

The water receiving tray 843 holds the gas-liquid contact member 841 from the lower side thereof, and it can stock electrolytic water passed through the gas-liquid contact member 841. A drain pipe 844 for guiding the electrolytic water to the drain pan 24 (see Figs. 2 and 3) is connected to the bottom surface of the water receiving tray 843.

Next, the operation of the air conditioning system 900 of the fifth embodiment will be described with reference to Fig. 13.

When an operation start instruction is input from an indoor remote controller (not shown) or the like by a user, the controller 863 switches the four-way valve 813 of the outdoor unit 801 to the cooling side or the heating side in accordance with the operation mode indicated by the operation start instruction to carry out a predetermined air conditioning operation such as a cooling operation, a heating operation or the like.

Here, under cooling operation, the controller 863 switches the four-way valve 813 to the cooling side, whereby refrigerant is made to flow into the refrigerant circuit 900b as indicated by an broken-line arrow of Fig. 13, the outdoor heat exchanger 814 serves as a condenser and the indoor heat exchanger 821 serves as an evaporator. Then, the air blowing fan 822 is operated to suck indoor air from the air suction port 831, the sucked indoor air is heat-exchanged by the indoor heat exchanger 821 and then the cooled air is supplied to the air filtering unit 804 in the indoor unit 802.

Under heating operation, the controller 863 switches the four-way valve 813 to the heating side, whereby the refrigerant flows into the refrigerant circuit 900b as indicated by a solid-line arrow of Fig. 13, the outdoor heat exchanger 814 serves as an evaporator and the indoor heat exchanger 821 serves as a condenser. Then, the air blowing fan 822 is operated to suck indoor air from the air suction port 831, the sucked air is heat-exchanged by the indoor heat exchanger 821 and then heated (warmed) air is supplied to the air filtering unit 804 in the indoor unit 802.

Under cooling or heating operation or under air blowing operation, an air filtering mode may be set in combination with each of these operation modes. When the air filtering mode is set, air-conditioned air is subjected to air filtering (i.e., inactivation, sterilization, decomposition, etc. of various bacteria, virus, fungus, etc. in air) in the air filtering unit 804, and then the filtered air is blown out to the room. In the following description, it is assumed that the air filtering mode is set.

As described above, simultaneously with the air-conditioning operation, the controller 863 controls supply of electrolytic water to the air filtering unit 804 as follows.

First, the controller 863 detects the water level in the electrolytic bath 861 by using the water level sensor 861c, and opens/closes the opening/closing valve 864a so that the water amount of tap water or the like in the electrolytic bath 861 is equal to a predetermined amount.

Then, the controller 863 detects the electrical conductivity in the electrolytic bath 861 by the electrode method using the electrodes 861a and 861b, and adjusts the opening degree of the flow rate (amount) adjusting valve 862c on the basis of the detected electric conductivity to control the addition amount of ion species so that the concentration of the ion species in the electrolytic bath 861 is optimal to electrolysis of tap water or the like.

Here, when ion species is added, the electrical conductivity of tap water or the like is detected because the concentration of the ion species in tap water or the like is detected to thereby determine the addition amount of the ion species which makes the concentration of the ion species in the electrolytic bath 861 optimal to electrolysis of tap water or the like. However, the relationship between the electrical conductivity in the electrical bath 861 and the concentration of ion species such as chloride ions or the like in the electrolytic bath 861 is determined by experiments or the like in advance and stored as data in ROM or the like equipped to the controller 863. For example, when the concentration of chloride ions in the electrolytic bath 861 is equal to 3000ppm, the electrical conductivity is equal to about 10000µS/cm or the like.

Current having a predetermined current density (for example, 20mA/cm² or the like) is supplied to the electrodes 861a, 861b for a predetermined time so that active oxygen species of a preset high concentration (for example, concentration of free residual chlorine of 30000mg/l or the like) is generated. Here, the concentration of the active oxygen species to be generated in the electrolytic bath 861 is set to about 100 times to 5,000 times of the concentration of the active oxygen species (2 to 10ppm in the case of the air filtering mode) contained in electrolytic water to be supplied to the air filtering unit 804.

The controller 863 adjusts the valve opening degree of the flow rate (amount) adjusting valve 865a to control the amount of the high concentration electrolytic water to be added to tap water or the like through the electrolytic water drop pipe 865, and electrolytic water containing a predetermined concentration of the active oxygen species is supplied to the air filtering unit 804.

However, it is assumed in the fifth embodiment that the water supply amount of tap water or the like in the water supply source connecting pipe (common water pipe) 851 is fixed. Furthermore, the relationship between the valve opening degree of the flow rate (amount) adjusting valve 865a and the concentration of the active oxygen species of electrolytic water to be supplied to the air filtering unit 804 is associated with each other by experiments or the like in advance, and the controller 863 controls the valve opening degree of the flow rate adjusting valve 865a so as to achieve the valve opening degree corresponding to the operation mode.

As described above, the electrolytic water in which the concentration of the active oxygen species is adjusted to a predetermined concentration is supplied from the water distributing pipe 852 to the air filtering nit 804 of each indoor unit 802. In the air filtering unit 804, the air supplied to the gas-liquid contact member 841 is brought into contact with the electrolytic water containing the active oxygen species to be filtered, and blown out from the air blow-out port 832 into the room.

The air conditioner 900a has a cleaning mode for cleaning the gas-liquid contact member 841. When the cleaning mode is set, the controller 863 adjusts the valve opening degree of the flow rate (amount) adjusting valve 865a so that electrolytic water in which the concentration of hypochlorous acid is adjusted to a predetermined value (for example, 100ppm) is supplied to the air filtering unit 804.

According to the fifth embodiment, air introduced into the indoor unit 802 by the air blowing fan 822 is heat-exchanged by the indoor heat exchanger 821 and then blown out to the room. Furthermore, air introduced into the indoor unit 802 by the air blowing fan 822 is brought into contact with electrolytic water containing active oxygen species and filtered in the air filtering unit 804. The air filtering unit 804 is supplied with electrolytic water containing a predetermined concentration of active oxygen species in accordance with the operation mode by the electrolytic water supply unit constructed by the electrolytic unit 806, the water distributing pipe 805, etc. In the electrolytic bath 861, high-concentration electrolytic water containing a higher concentration of active oxygen species than electrolytic water to be supplied to the air filtering unit 804 is generated and added to water supplied from an external water supply source so that the concentration of the active oxygen species is adjusted to a predetermined concentration, and then the adjusted electrolytic water is supplied to the air filtering unit 804.

Here, in the above fifth embodiment, electrolytic water containing active oxygen species whose concentration is equal to about 100 times to 5, 000 times of the concentration of active oxygen species contained in electrolytic water to be supplied to the air filtering unit 804 is generated in the electrolytic unit 806. As a method of generating high-concentration electrolytic water in the electrolytic unit 806 can be used a method of increasing current flowing between the electrodes 861a and 8 61b to increase the current density and a method of lengthening the electrolysis time when the water quality of tap water or the like to be introduced into the electrolytic bath 861 is fixed. However, when the current density is increased, the electrodes are intensively worn, and the lifetime of the electrodes may be shortened. On the other hand, when the electrolysis time is lengthened, the time period until the time reaches the lifetime of the electrodes is shortened. Therefore, when tap water or the like which is rare in chloride ions is introduced into the electrolytic bath 861 or the like, in consideration of the lifetime of the electrodes 861a, 861b, generation of the concentration of generable active oxygen species, that is, electrolytic water containing high-concentration active oxygen species is restricted in accordance with the water quality of tap water or the like supplied to electrolysis. However, in the fifth embodiment, the ion species adding unit 862 is provided to add a predetermined ion species into the electrolytic bath 861, and the concentration of the ion species of tap water or the like introduced into the electrolytic bath 861 is adjusted. Accordingly, the generation efficiency of the ion species required to generate the active oxygen species is increased, and occurrence of oxygen and hydrogen in the electrolysis process can be reduced, and the electrolysis time can be shortened, so that power consumption can be reduced.

Furthermore, in a case where the concentration of active oxygen species contained in electrolytic water supplied to the air filtering unit 804 is adjusted to a desired concentration, the process of generating electrolytic water to be supplied to the air filtering unit 804 does not adopt a method of adjusting the concentration of active oxygen species generated simultaneously with generation of electrolytic water in the electrolytic bath 861, but a method of diluting high-concentration electrolytic water containing high-concentration active oxygen species generated in the electrolytic bath 861 with water supplied from the water supply source, thereby adjusting the concentration of the active oxygen species. Therefore, in the electrolytic unit 806, even when the concentration of the active oxygen species to be supplied to the air filtering unit 804 is required to be changed, the concentration of the active oxygen species in the electrolytic water generated in the electrolytic unit 806 is merely set to a preset concentration at the electrolytic unit 806 side. Therefore, the concentration management for generation of electrolytic water is easy. Furthermore, the concentration of the active oxygen species can be easily adjusted to a desired concentration by merely changing the mixing ratio of the electrolytic water generated in the electrolytic unit 806 and water supplied from the water supply source.

As in the case of the first to fourth embodiments, as a result of a verification test for this embodiment which was made in cooperation with Sanitary Environment Research, it has been found that when air in which influenza virus invades is passed through the gas-liquid contact member 841 of this embodiment, 99% or more of the virus concerned can be removed.

Furthermore, in the fifth embodiment, the water distributing pipe 805 is constructed by the common pipe 851 connected to the water pipe and the water distributing pipe 852 which is branched from the common pipe 851 to distribute water to each indoor unit, and the electrolytic unit 806 collectively adds high-concentration electrolytic water to tap water or the like from the water supply source through the common pipe 851. Therefore, it is unnecessary to provide the electrolytic unit 806 every indoor unit 802, and the construction of the air conditioning system 900 and the air conditioner 900a can be simplified.

Furthermore, as in the case of the air conditioning system 900 of the fifth embodiment, the air flowing passage extending from the air suction port 831 to the air blow-out ports 832 is formed by the air flowing fan 822, and the air filtering unit 804 is disposed at the downstream side of the indoor heat exchanger 821 on the air flowing passage, whereby the following effects can be achieved.

That is, under cooling operation of the air conditioning system 900 of the fifth embodiment, air which is cooled by the indoor heat exchanger 821 and thus has relatively high humidity is supplied to the air filtering unit 804. Under heating operation, air which is heated by the indoor heat exchanger 821 and thus has relatively low humidity is supplied to the air filtering unit 804. Accordingly, even when the supplied air is brought into contact with electrolytic water in the air filtering unit 804, the air passed from the air filtering unit (i.e., after the air filtering) can be suppressed from increasing in relatively humidity because the air having relatively high humidity is supplied to the air filtering unit under cooling operation. Furthermore, under heating operation, air having low relative humidity is supplied to the air filtering unit 804, and thus the relative humidity of the air after the air filtering can be increased by bringing the air into contact with the electrolytic water in the air filtering unit 804. Accordingly, the humidification amount under air-conditioning operation can be automatically controlled without increasing the air-conditioning load by merely carrying out the air filtering/cleaning operation in a wet style and switching the four-way valve 813 to switch the cooling operation and the heating operation to each other, whereby the air environment of the room can be kept comfortable.

Furthermore, under cooling operation, the heat-exchanged air is filtered in the air filtering unit 804, and thus clean air can be supplied to the room. In addition, the air supplied to the air filtering unit has relatively high humidity, and thus consumption of electrolytic water in the air filtering unit can be suppressed.

Still furthermore, according to the fifth embodiment, electrolytic water containing active oxygen species such as hypochlorous acid or the like is discharged from the water receiving tray 843 below the air filtering unit 804 through the drain pipe 844 to the drain pan 824. Therefore, drain water stocked in the drain pan 824 is contaminated with electrolytic water to thereby prevent occurrence of various bacteria, virus, etc. in the drain water and thus prevent occurrence of slime on the drain pan 824. Therefore, the cleaning and maintenance frequency of the drain pan 824 is reduced, and thus the labor of the cleaning and maintenance works can be reduced.

According to the fifth embodiment, the air filtering unit 804 is provided to the air blow-out port 832 side of the indoor unit 802,

Furthermore, according to the fifth embodiment, the air filtering unit 804 is provided to the air blow-out port 832 side of the indoor unit 802, and thus the active oxygen species of hypochlorous acid or the like contained in air blown out from the air filtering unit 804 is not directly introduced to the indoor heat exchanger 821. Therefore, the indoor heat exchanger 821 can be prevented from being corroded by the active oxygen species such as hypochlorous acid or the like.

The present invention is not limited to the fifth embodiment described above, and various modifications may be made without departing from the subject matter of the present invention.

For example, in the fifth embodiment, the valve opening degree of the flow rate (amount) adjusting valve 865a is adjusted to control the amount of high-concentration electrolytic water added to tap water or the like through the electrolytic water drop pipe 865, whereby the concentration of active oxygen species contained in electrolytic water supplied to air filtering unit 804 is adjusted to a desired concentration. In place of this method, the following method may be adopted. That is, the drop amount of the high-concentration electrolytic water is set to be fixed irrespective of the operation mode or the like, the flow rate (amount) adjusting valve is provided to the common pipe 851 and the water supply amount of tap water or the like supplied from the water supply source is varied, whereby the mixing ratio of the high-concentration electrolytic water and tap water or the like is changed, thereby adjusting the concentration of active oxygen species in electrolytic water.

Furthermore, when ion species is added, the electrical conductivity in the electrolytic bath 861 is detected, and the ion species is added so that the concentration of the ion species in the electrolytic bath 861 is optimal. However, when the concentration variation of ion species such as chloride ions or the like (variation of electrical conductivity) is little, the concentration of the ion species concerned may be measured in advance when the apparatus is set up, and the valve opening corresponding to the concentration concerned may be preset. In this construction, the detection of the electrical conductivity of tap water or the like may be carried out when the electrolysis of tap water or the like is started. However, since the electrical conductivity is not greatly varied in a day, and thus it may be detected every time, but once every several times of electrolysis.

The fifth embodiment relates to a so-called in-ceiling embedded type and four-wayblow-out type indoor unit 802. However, the present invention is not limited to this type of indoor unit, and it may be a ceiling-suspended type, a wall-suspended type or an on-floor mount type. Furthermore, the air filtering unit 804 is disposed between the thermal insulting member 823 and one side of the indoor heat exchanger 821 which is disposed in the housing 820 of the indoor unit 802 and bent in a substantially rectangular shape. However, the air filtering unit 804 may be disposed along one or more sides of the indoor heat exchanger 821. The gas-liquid contact member 841 of the air filtering unit 804 may be disposed in parallel to or obliquely to the indoor heat exchanger 821.

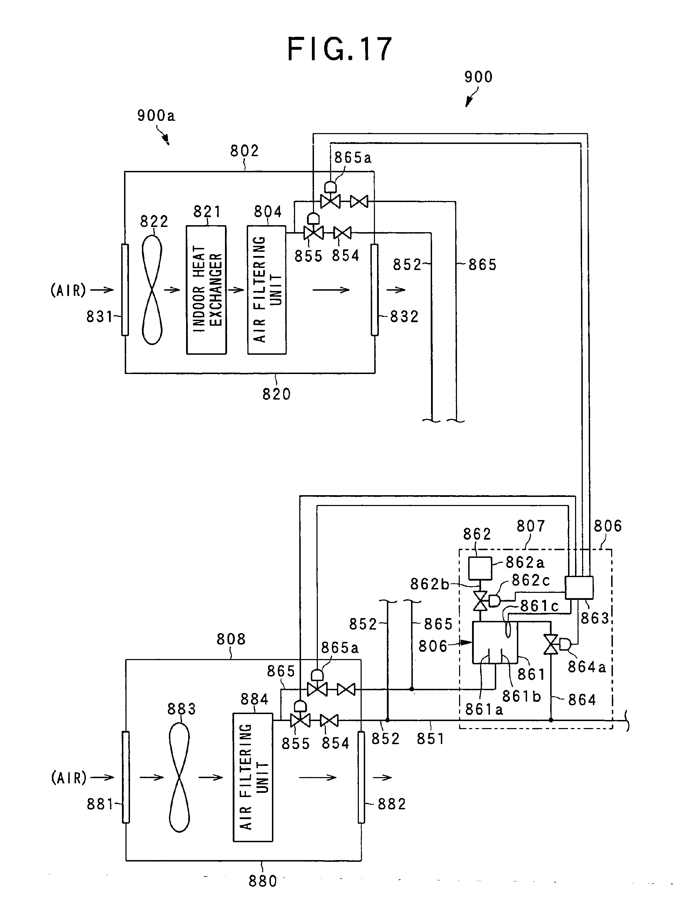

In the fifth embodiment, the air conditioning system 900 includes the air conditioner 900a in which one or plural indoor units 802 each of which includes an air filtering unit 804 are provided in connection with one outdoor unit 801. In addition to this type of air conditioner 900a, the air conditioning system 900 may be further equipped with one or more air filtering apparatuses 808 as shown in Fig. 17.

The air filtering apparatus shown in Fig. 21 includes a housing 880 having an air suction port 881 and an air blow-out port 882, an air blowing fan 883 for blowing air sucked from the air suction port 881 to the air blow-out port 882, and an air filtering unit 884 which is disposed on an air flowing passage formed in the housing 880 by the air blowing fan 883 and filters air by bringing air supplied through the air blowing passage into contact with electrolytic water containing active oxygen species. However, in Fig. 17, the same constituent elements as the fifth embodiment are represented by the same reference numerals.

In the case of Fig. 17, the water distributing pipe 852 branched from the common pipe 851 is also connected to the air filtering unit 884 of the air filtering apparatus 808, and electrolytic water is supplied from the electrolytic unit 806 through the water distributing pipe 852. The flow rate (amount) adjusting valve 855 is provided to each water distributing pipe 852 to adjust the flow rate (amount) of tap water or the like supplied from the water supply source

The electrolytic water drop pipe 865 is designed so that high-concentration electrolytic water is not dropped to the common pipe 851, but also dropped to each water distributing pipe 852. Therefore, the flow rate (amount) adjusting valve 865a is provided to each of the indoor units 802 and the air filtering apparatus 808 so that the drop amount of the high-concentration electrolytic water to each of the indoor units 802 and the air filtering apparatus 808 can be individually controlled. Therefore, the drop amount of the high-concentration electrolytic water and the water supply amount from the water supply source are controlled individually for each of the indoor units 802 and the air filtering apparatus 808, whereby electrolytic water in which electrolytic water can be supplied to each of the air filtering units 804, 884 while the concentration of the active oxygen species of the electrolytic water is adjusted to desired different concentrations between the air filtering units 804 and 884.

According to the construction shown in Fig. 17, when electrolytic water is supplied to each of the air filtering units 804 and 885 of the plural indoor units 802 and the air filtering apparatus 808, high-concentration electrolytic water can be efficiently generated in the electrolytic bath 861, and the high-concentration electrolytic water is diluted with tap water or the like, whereby electrolytic water containing a predetermined concentration of active oxygen species can be supplied to each of the air filtering units 804 and 885. Furthermore, even when the concentration of active oxygen species of electrolytic water to be supplied to each indoor unit 802 or the air filtering apparatus 808 is changed in accordance with the operationmode or the like, the electrolytic water containing an optimal concentration of active oxygen species can be easily prepared by controlling the drop amount of the high-concentration electrolytic water and the supply amount of tap water or the like to change the mixing ratio therebewteen. Accordingly, it is unnecessary to individually provide the electrolytic unit 806 for each of the indoor units 802 and the air filtering apparatus 808, and thus the construction of the system can be simplified.

Furthermore, in the air conditioning system 900 (air filtering system) according to the present invention, in place of or in addition to some or all of the indoor units 802, plural air filtering apparatuses 808 may be provided. In this case, the air filtering unit 884 and the electrolytic unit 885 provided to the air filtering apparatus 808 may be designed in the same construction as the air filtering unit 804 and the air filtering unit 804 provided to the indoor unit 802.

Still furthermore, only one indoor unit 802 maybe provided, or only one air filtering apparatus 808 may be provided.

When only one indoor unit 802 is provided or only one air filtering apparatus 808 is provided, the electrolytic unit 806 may be accommodated in the housing 820, 880.

In the above fifth embodiment, hypochlorous acid is generated as the active oxygen species. However, in place of hypochlorous acid, ozone (O₃) or hydrogen peroxide (H₂O₂) may be generated as the active oxygen species. In this case, if platinum tantalum electrodes are used as the electrodes in the electrolytic unit 405, the active oxygen species can be highly efficiently and stably generated from even ion-species rare water by electrolysis.

At this time, at the anode, the following reaction occurs:

2H₂O → 4H⁺ + O₂ + 4e⁻

At the same time, the following reactions occur, and ozone (O₃) is generated:

3H₂O → O₃ + 6H⁺ + 6e⁻

2H₂O → O₃ + 4H⁺ + 4e⁻

At the cathode, O₂⁻ generated through the electrode reaction and H⁺ in the solution react with each other, and hydrogen peroxide (H₂O₂) is generated according to the following reactions:

4H⁺ + 4e⁻ + (4OH) → 2H₂ + (4OH)

O₂⁻ + e⁻ + 2H⁺ → H₂O₂

In this construction, by supplying current to the electrodes 861a, 861b, ozone (O₃) and hydrogen peroxide (H₂O₂) having strong sterilizing power occur, and electrolytic water containing ozone (O₃) and hydrogen peroxide (H₂O₂) is generated. The concentration of ozone or hydrogen peroxide in the electrolytic water is adjusted to such a concentration that target virus, etc. can be inactivated, and air is passed through the gas-liquid contact member 841 supplied with the electrolytic water of this concentration, whereby the target virus, etc. floatingintheaircanbe inactivated. Furthermore, when gaseous material such as odor or the like passes through the gas-liquid contact member 841, it is dissolved in the electrolytic water or it reacts with ozone or hydrogen peroxide in the electrolytic water, so that these materials are removed from the air and thus deodorized.

When scales are deposited on the electrodes 861a, 861b (cathode) through the electrolysis of tap water or the like, the electrical conductivity is lowered and the water flow to the electrolytic plane is disturbed, so that it is difficult to continuously perform electrolysis. In this case, it is effective to invert the polarities of the electrodes 861a, 861b (the plus and minus of the electrodes 861a, 861b are switched to each other). By conducting electrolysis while the cathode is set to the anode, the scales deposited on the cathode can be removed. In this polarity inverting control, for example, the polarities may be inverted periodically by using a timer or irregularly inverted by inverting the polarities every time the operation is started, for'example. Furthermore, increase of the electrolysis resistance (reduction in electrolysis current or increase of electrolytic voltage) may be detected, and the polarities may be inverted on the basis of the detection result.

## Claims

1. An air conditioner having a refrigerant circuit including a compressor, a four-way valve, an outdoor heat exchanger, an air blower for sucking air and blowing out the air to a room, and an indoor heat exchanger for heat-exchanging the air sucked by the air blower, the indoor heat exchanger and the air blower being equipped in a housing of an indoor unit, **characterized by** comprising:
an air filtering unit that is disposed in the indoor unit to bring the air sucked by the air blower into contact with electrolytic water containing active oxygen species sucked to filter the air; and
an electrolytic water supply unit for electrolyzing water containing predetermined ion species to generate electrolytic water containing active oxygen species of a prescribed concentration and supplying the generated electrolytic water to the air filtering unit.

2. The air conditioner according to claim 1, wherein the indoor unit further includes a bypass flow passage that is disposed between the air filtering unit and the electrolytic water supply unit and through which foreign materials from the electrolytic water supply unit are selectively removed.

3. The air conditioner according to claim 2, wherein the foreign materials are scales occurring due to generation of the electrolytic water.

4. The air conditioner according to claim 2, wherein the indoor unit includes a main flow passage through which the electrolytic water is supplied from the electrolytic water supply unit to the air filtering unit, and the bypass flow passage is branched from the main flow passage at some position between the air filtering unit and the electrolytic water supply unit.

5. The air conditioner according to claim 4, wherein the electrolytic water supply passage is equipped with an opening/closing valve that is closed to prevent the foreign materials from flowing through the electrolytic water supply passage to the air filtering unit when the foreign materials are removed, and the bypass flow passage is equipped with an opening/closing valve that is closed to prevent the electrolytic water from flowing through the bypass flowpassage when the foreign materials are not removed.

6. The air conditioner according to claim 2, wherein the indoor unit further includes a drain pan disposed below the heat exchanger and a water receiving tray disposed below the air filtering unit, and the bypass flow passage is connected to at least one of the drain pan and the water receiving tray to discharge the foreign materials from the electrolytic water supply unit to at least one of the drain pan and the water receiving tray.

7. The air conditioner according to claim 1, wherein the electrolytic water supply unit has at least one pair of electrodes for electrolyzing water, and scales occurring on the electrodes through the electrolysis of water are removed by inverting the polarities of the electrodes.

8. The air conditioner according to claim 1, further comprising a controller for controlling a process of generating the electrolytic water in the electrolytic water supply unit, wherein on the basis of a control signal from the controller, the electrolyticwatersupplyunitgenerateselectrolyticwaterwhose concentration is higher than that of the electrolytic water used for air filtering, and supplies the higher-concentration electrolytic water to the air filtering unit.

9. The air conditioner according to claim 8, wherein the higher-concentration electric water is supplied from the electrolytic water supply unit to the air filtering unit under the state that the operation of the air blower is stopped.

10. The air conditioner according to claim 8, wherein a cleaning operation mode is provided as one of operation modes of the indoor unit, and the controller controls the electrolytic water supply unit to supply the higher-concentration electrolytic water to the air filtering unit for a predetermined time or at a predetermined time interval in the cleaning operation mode.

11. The air conditioner according to claim 10, wherein the cleaning operation mode is a mode in which the higher-concentration electrolytic water is supplied to the air filtering unit at the predetermined time interval while the operation of the indoor unit is stopped.

12. The air conditioner according to claim 10, wherein the cleaning operation mode is a mode in which the higher-concentration electrolytic water is supplied to the air filtering unit for the predetermined time while the indoor unit is continuously operated.

13. The air conditioner according to claim 8, wherein the controller controls the electrolytic water supply unit to change a current value for electrolysis, thereby adjusting the concentration of the electrolytic water determined on the basis of the current value.

14. The air conditioner according to claim 1, wherein the air filtering unit is equipped with a gas-liquid contact member at which the indoor air and the electrolytic water are brought into contact with each other, and a water receiving tray for temporarily stocking the electrolytic water dropped from the gas-liquid contact member, and a circulating pump for pumping up the electrolytic water stocked in the water receiving tray and circulating the electrolytic water concerned to the gas-liquid contact member.

15. The air conditioner according to claim 14, further comprising a drain pan that is disposed below the heat exchanger and stocks drain water dropped from the heat exchanger, and a passage through which the electrolytic water stocked in the water receiving tray is introduced from the water receiving tray to the drain pan.

16. The air conditioner according to claim 15, further comprising an electromagnetic valve that is provided in the passage and opened/closed to adjust the amount of the electrolytic water to be introduced from the water receiving tray to the drain pan.

17. The air conditioner according to claim 15, wherein the electromagnetic valveisintermittently opened/closedto thereby intermittently introduce the electrolytic water stocked in the water receiving tray to the drain pan.

18. The air conditioner according to claim 15, further comprising a dam portion provided in the passage, wherein electrolytic water flowing over the dam portion is introduced to the drain pan.

19. The air conditioner according to claim 14 , further comprising a water level detecting unit for detecting whether the electrolytic water stocked in the electrolytic receiving tray is equal to a predetermined water level or less, and a water supply unit for supplying one of water or water containing the predetermined ion species.

20. The air conditioner according to claim 19, wherein the predetermined ion species is chlorine ion.

21. The air conditioner according to claim 1, further comprising an ion species adding unit for adding the predetermined ion species to water supplied from an external water supply source to the electrolytic water supply unit.

22. The air conditioner according to claim 21, wherein the ion species adding unit is disposed in a water distributing passage of a water distributing pipe through which the electrolytic water supply unit and an external water supply source are connected to each other, and adds the predetermined ion species to water which is supplied from the external water supply source to the electrolytic water supply unit.

23. The air conditioner according to claim 22, wherein the ion species adding unit comprises a stock tank for stocking material containing the predetermined ion species, a distributing pipe through which the stock tank and the water distributing pipe are connected to each other, an electrical conductivity detecting unit for detecting the electrical conductivity of water supplied from the water supply source, and a control unit for controlling the amount of the predetermined ion species to be added from the water supply source to the water supplied to the electrolytic water supply unit on the basis of the electrical conductivity detected by the electrical conductivity detecting unit.

24. The air conditioner according to claim 23, wherein the control unit adds the predetermined ion species to the water when the electrical conductivity detected by the electrical conductivity detecting unit is lower than a predetermined value.

25. The air conditioner according to claim 23, wherein the ion species adding unit further comprises an adjusting valve that is provided to the distributing pipe and adjusts the amount of the predetermined ion species to be introduced from the stock tank into the water distributing pipe, and the control unit adjusts the valve opening degree of the adjusting valve to control the amount of the predetermined ion species to be added from the water supply source to the electrolytic water supply unit.

26. The air conditioner according to claim 23, wherein the stock tank is disposed at a low place in the room and connected to the water distributing pipe through the distributing pipe.

27. The air conditioner according to claim 1, wherein the ion species is halide ion.

28. The air conditioner according to claim 1, wherein the active oxygen species is at least one material selected from the group consisting of hypochlorous acid, ozone and hydrogen peroxide.

29. The air conditioner according to claim 1, wherein the electrolytic water supply unit has a mixing unit for generating electrolytic water having a higher concentration than the concentration of electrolytic water supplied to the air filtering unit under a normal air filtering operation, and adding the higher-concentration electrolytic water concerned to water supplied from an external water supply source so that the concentration of the active oxygen species of the generated electrolytic water is equal to the prescribed concentration of the active oxygen species of the electrolytic water supplied to the air filtering unit.

30. The air conditioner according to claim 1, wherein the electrolytic water supply unit is equipped to the housing of the indoor unit.

31. An air conditioning system equipped with an outdoor unit having a compressor, a four-way valve and an outdoor heat exchanger, and one or more indoor units each having an air blower for sucking air and blowing out the air to a room and an indoor heat exchanger for heat-exchanging the air sucked by the air blower, the indoor heat exchanger and the air blower being equipped in the housing of each indoor unit, **characterized in that** each indoor unit has an air filtering unit for bringing the air sucked by the air blower into contact with electrolytic water containing active oxygen species sucked to filter the air, and an electrolytic water supply unit for electrolyzing water containing predetermined ion species to generate electrolytic water containing active oxygen species of a prescribed concentration is connected to the plural indoor units and supplies the generated electrolytic water to the air filtering units of the plural indoor units.

32. The air conditioning system according to claim 31, wherein the electrolytic water supply unit generates electrolytic water having a higher concentration than the concentration of electrolytic water supplied to the air filtering unit under a normal air filtering operation, and adds the higher-concentration electrolytic water concerned to water supplied from an external water supply source so that the concentration of the active oxygen species of the generated electrolytic water is equal to the prescribed concentration of the active oxygen species of the electrolytic water supplied to the air filtering unit.

33. The air conditioning system according to claim 32, wherein the electrolytic water supply unit has a concentration adjusting unit for controlling the amount of the higher-concentration electrolytic water to be added to the water supplied from the water supply source to thereby adjust the concentration of the active oxygen species of the electrolytic water supplied to the air filtering unit.

34. The air conditioning system according to claim 32, wherein the electrolytic water supply unit has a concentration adjusting unit for controlling the flow rate of the water supplied from the water supply source when the higher-concentration electrolytic water is added to the water, thereby adjusting the concentration of the active oxygen species of the electrolytic water supplied to the air filtering unit.

35. The air conditioning system according to claim 32, wherein the electrolytic water supply unit has a concentration adjusting unit for controlling the addition amount of the higher-concentration electrolytic water to the water which is supplied from the water supply source to the air filtering unit of each indoor unit, thereby adjusting the concentration of the active oxygen species of the electrolytic water supplied to each air filtering unit every air filtering unit.

36. The air conditioning system according to claim 32, wherein the electrolytic water supply unit has a concentration adjusting unit for controlling the flow rate of the water supplied from the power supply source to the air filtering unit of each indoor unit, thereby adjusting the concentration of the active oxygen species of the electrolytic water supplied to each air filtering unit every air filtering unit.

37. The air conditioning system according to claim 32, wherein the electrolytic water supply unit has at least a pair of electrodes, an electrolytic water bath to which water is introduced from the water supply source and in which the introduced water is electrolyzed, and an ion species adding unit for adding the predetermined ion species to the water introduced to the electrolytic bath.

38. The air conditioning system according to claim 37, wherein the ion species adding unit comprises an electrical conductivity detecting unit for detecting the electrical conductivity of the water introduced into the electrolytic water, and a controller for controlling the amount of the ion species added to the water introduced into the electrolytic water bath on the basis of the electrical conductivity detected by the electrical conductivity detecting unit.

39. The air conditioning system according to claim 31, wherein the ion species is halide ion.

40. The air conditioning system according to claim 31, wherein the active oxygen species is at least one material selected from the group consisting of hypochlorous acid, ozone and hydrogen peroxide.

41. The air conditioning system according to claim 32, wherein the housing has an air suction port through which air is sucked and an air blow-out portion through which air is blown out to the room, the air blower forms an air flowing passage in the housing so that the air sucked from the air suction port flows to the air blow-out port through the air flowing passage, and the air filtering unit is disposed on the air flowing passage to bring the air supplied through the air flowing passage into contact with the electrolytic water containing the predetermined concentration of the active oxygen species to filter the air.

42. The air conditioning system according to claim 31, further comprising at least one air filtering apparatus including an air blower for sucking air and blowing out the air to the room and an air filtering unit for bringing air sucked by the air blower into contact with electrolytic water containing active oxygen species to filter the air, and the air filtering apparatus is supplied with the electrolytic water from the electrolytic water supply unit.

43. An air conditioning system equipped with an outdoor unit having a compressor, a four-way valve and an outdoor heat exchanger, and one or more indoor units each having an air blower for sucking air and blowing out the air to a room and an indoor heat exchanger for heat-exchanging the air sucked by the air blower, the indoor heat exchanger and the air blower being equipped in the housing of each indoor unit, **characterized in that** each indoor unit is equipped with an air filtering unit for bringing the air sucked by the air blower into contact with electrolytic water containing active oxygen species sucked to filter the air, and an electrolytic water supply unit for electrolyzing water containing predetermined ion species to generate electrolytic water containing active oxygen species of a prescribed concentration and supplies the generated electrolytic water to the air filtering unit, and the air conditioning system is further equipped with a water distributing pipe through which the electrolytic water supply unit of each indoor unit is connected to an external water supply source to supply water from the external water supply source to the electrolytic water supply unit, and an ion species adding unit that is equipped in a water distributing passage formed by the water distributing pipe, adds the ion species to the water supplied from the water supply source to the electrolytic water supply unit and supplies the water added with the ion species to the electrolytic water supply units of the indoor units.

44. The air conditioning system according to claim 43, wherein the water distributing pipe comprises a common pipe portion connected to the water supply source and distributing pipe portions that are branched from the common pipe portion and connected to the respective electrolytic water supply units of the corresponding indoor units to supply water to the indoor units, and the ion species adding unit is disposed in a water distributing passage formed by the common distributing pipe portion.

45. The air conditioning system according to claim 43, further comprising at least one air filtering apparatus including an air blower for sucking air and blowing out the air to the room, an air filtering unit for bringing air sucked by the air blower into contact with electrolytic water containing active oxygen species to filter the air, and an electrolytic water supply unit for electrolyzing water containing ion species to generate electrolytic water containing active oxygen species and supplies the generated electrolytic water to the air filtering unit, wherein the electrolytic water supply unit is supplied with the water added with the ion species from the ion species adding unit.

46. An air filtering apparatus comprising:
a housing having an air suction port through which air is sucked and an air blow-out port through which air is blown out to a room;
an air blowing fan that is disposed in the housing and makes the air sucked from the air suction port flow to the air blow-out port;
an air filtering unit that is disposed in an air flowing passage formed in the housing by the air blowing fan and brings the air supplied through the air flowing passage into contact with electrolytic water containing a predetermined concentration of active oxygen species to filter the air; and
an electrolytic water supply unit for generating higher-concentration electrolytic water containing active oxygen species whose concentration is higher than the predetermined concentration, adding the higher-concentration electrolytic water with water supplied from an external water supply source so as to achieve the electrolytic water containing the active oxygen species having the predetermined concentration, and supplying the electrolytic water concerned to the air filtering unit.

47. An air filtering apparatus comprising:
a housing having an air suction port through which air is sucked and an air blow-out port through which air is blown out to a room;
an air blowing fan that is disposed in the housing and makes the air sucked from the air suction port flow to the air blow-out port;
an air filtering unit that is disposed in an air flowing passage formed in the housing by the air blowing fan and brings the air supplied through the air flowing passage into contact with electrolytic water containing a predetermined concentration of active oxygen species to filter the air;
an electrolytic water supply unit for electrolyzing water containing a predetermined ion species to generate electrolytic water containing the active oxygen species and supplying the generated electrolytic water to the air filtering unit; and
an ion species adding unit for adding the ion species to water supplied from an external water supply source to the electrolytic water supply unit.

48. An air filtering system comprising:
one or more air filtering apparatuses for filtering indoor air, each air filtering apparatus including a housing having an air suction port through which air is sucked and an air blow-out port through which air is blown out to a room, an air blowing fan that is disposed in the housing and makes the air sucked from the air suction port flow to the air blow-out port, an air filtering unit that is disposed in an air flowing passage formed in the housing by the air blowing fan and brings the air supplied through the air flowing passage into contact with electrolytic water containing a predetermined concentration of active oxygen species to filter the air, and an electrolytic water supply unit for electrolyzing water containing a predetermined ion species to generate electrolytic water containing the active oxygen species and supplying the generated electrolytic water to the air filtering unit;
a water distributing pipe including a common water distributing pipe portion connected to an external water supply source and one or more distributingpipe portions that are branched from the common water distributing pipe portion and connected to the electrolytic water supply units of the respective indoor units to distribute water to the electrolytic water supply units of the respective indoor units; and
an ion species adding unit that is disposed in a water distributing passage formed by the common water distributing pipe portion and adds the ion species to the water supplied from the external water supply source.

49. An air filtering system comprising:
one or more air filtering apparatuses for filtering indoor air, each air filtering apparatus comprising a housing having an air suction port through which air is sucked and an air blow-out port through which air is blown out to a room, an air blowing fan that is disposed in the housing and makes the air sucked from the air suction port flow to the air blow-out port, and an air filtering unit that is disposed in an air flowing passage formed in the housing by the air blowing fan and brings the air supplied through the air flowing passage into contact with electrolytic water containing a predetermined concentration of active oxygen species to filter the air; and
an electrolytic water supply unit for generating higher-concentration electrolytic water containing active oxygen species whose concentration is higher than the predetermined concentration, adding the higher-concentration electrolytic water with water supplied from an external water supply source so as to achieve the electrolytic water containing the active oxygen species having the predetermined concentration, and supplying the electrolytic water concerned to the respective air filtering units.
